# EUROPEAN PATENT APPLICATION

(11) **EP 4 806 883 A1**
(43) Date of publication of application: **16.09.2026**
(21) Application number: 24888706.9
(22) Date of filing: 05.11.2024
(51) Int. Cl.: C07C 321/14, C08G 18/38, C08J 5/00, G02B 1/04, G02B 3/00, C07C 319/14, C07C 319/28

(54) **POLYTHIOL COMPOSITION AND APPLICATION THEREOF**

(30) Priority: 09.11.2023 JP 2023191755; 11.06.2024 JP 2024094653
(71) Applicant: Mitsui Chemicals, Inc., Tokyo 104-0028 (JP)
(72) Inventor: NAKANO, Shotaro, Omuta-shi, Fukuoka 836-8610 (JP)
(74) Representative: Mewburn Ellis LLP
(86) International application number: PCT/JP2024/039310
(87) International publication number: WO 2025/100411

(57) **Abstract**

A polythiol composition, including: a polythiol component T, which is at least one selected from the group consisting of 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane; a component X, which has a retention time of from 30.0 minutes to 40.0 minutes in a specified HPLC; and a component Y, for which the retention time is from 60.0 minutes to 70.0 minutes, wherein, in the specified HPLC, the component X has a peak area of 5.00 or less with respect to the total peak area of 100, and the component Y has a peak area of 2.50 or less with respect to the total peak area of 100.

## Description

### Technical Field

The present disclosure relates to a polythiol composition and application thereof.

### Background Art

Plastic lenses, which are resin-containing lenses, are lightweight, hardly breakable, and dyeable as compared to inorganic lenses; therefore, in recent years, the use of plastic lenses as eyeglass lenses, camera lenses, and the like has been rapidly increased.

For example, Patent Documents 1 and 2 disclose tetrafunctional thiols having specific structures as raw materials for plastic lenses.

Patent Document 1: Japanese Patent Application Laid-Open (JP-A) No. H7-252207
Patent Document 2: WO2014/027428

### SUMMARY OF THE INVENTION

### Technical Problem

Examples of the tetrafunctional thiols disclosed in Patent Documents 1 and 2 include three compounds, which are 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane.

However, in a resin produced using at least one selected from the group consisting of these three compounds (hereinafter, also referred to as "polythiol component T"), a reduction of the yellowness and/or an improvement of the heat resistance may be required.

An object of the first and the second embodiments of the disclosure is to provide: a polythiol composition from which a resin with reduced yellowness and improved heat resistance can be produced; and application thereof.

### Solution to Problem

Means for solving the above-described problems encompass the following aspects.

The polythiol composition according to the first embodiment of the disclosure is the polythiol composition according to <1> below.

The polythiol composition according to the second embodiment of the disclosure is the polythiol composition according to <5> below.
<1> A polythiol composition, comprising:
   a polythiol component T, which is at least one selected from the group consisting of 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane;
   a component X, which has a retention time of from 30.0 minutes to 40.0 minutes in a high-performance liquid chromatography measurement performed under the following measurement conditions A; and
   a component Y, for which the retention time is from 60.0 minutes to 70.0 minutes,
   wherein, in the high-performance liquid chromatography measurement, the polythiol component T has a peak area of 80.00 or more with respect to a total peak area of 100 of compounds contained in the polythiol composition, the component X has a peak area of 5.00 or less with respect to the total peak area of 100, and the component Y has a peak area of 2.50 or less with respect to the total peak area of 100:
      - Measurement Conditions A-
   YMC-Pack (registered trademark) ODS-A manufactured by YMC Co., Ltd. (particle size S: 5 µm, column shape: Φ6 mm × 150 mm) is used as a column;
   a mixed solution of acetonitrile / 0.01mol/L-aqueous potassium dihydrogen phosphate solution = 60/40 (vol/vol) is used as a mobile phase;
   a mixed solution of 160 mg of the polythiol composition and 10 mL of acetonitrile is used as a measurement solution;
   a UV detector having a measurement wavelength of 230 nm is used as a detector;
   a column temperature is set at 40°C;
   a flow rate is set at 1.0 mL/min; and
   an injection amount is set at 2 µL.
<2> The polythiol composition according to <1>, wherein:
   the component X is a polythiol compound having a molecular weight of 426, and
   the component Y is a polythiol compound having a molecular weight of 532.
<3> The polythiol composition according to <1> or <2>, wherein:
   the peak area of the component X is 1.00 or more, and
   the peak area of the component Y is 1.00 or more.
<4> The polythiol composition according to any one of <1> to <3>, wherein a ratio of the peak area of the component Y to the peak area of the component X is from 0.40 to 0.90.
<5> A polythiol composition, comprising:
   a polythiol component T, which is at least one selected from the group consisting of 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane;
   a component W, which has a retention time of from 8.7 minutes to 9.7 minutes in a high-performance liquid chromatography measurement performed under the following measurement conditions A; and
   a component V, for which the retention time is from 11.0 minutes to 13.5 minutes,
   wherein, in the high-performance liquid chromatography measurement, the polythiol component T has a peak area of 75.00 or more with respect to a total peak area of 100 of compounds contained in the polythiol composition, the component W has a peak area of 3.50 or less with respect to the total peak area of 100, and the component V has a peak area of 3.10 or less with respect to the total peak area of 100:
      - Measurement Conditions A-
   YMC-Pack (registered trademark) ODS-A manufactured by YMC Co., Ltd. (particle size S: 5 µm, column shape: Φ6 mm × 150 mm) is used as a column;
   a mixed solution of acetonitrile / 0.01mol/L-aqueous potassium dihydrogen phosphate solution = 60/40 (vol/vol) is used as a mobile phase;
   a mixed solution of 160 mg of the polythiol composition and 10 mL of acetonitrile is used as a measurement solution;
   a UV detector having a measurement wavelength of 230 nm is used as a detector;
   a column temperature is set at 40°C;
   a flow rate is set at 1.0 mL/min; and
   an injection amount is set at 2 µL.
<6> The polythiol composition according to <5>, wherein:
   the component W is a polythiol compound having a molecular weight of 350, and
   the component V is a polythiol compound having a molecular weight of 260.
<7> The polythiol composition according to <5> or <6>, wherein:
   the peak area of the component W is 1.30 or more with respect to the total peak area of 100, and
   the peak area of the component V is 1.00 or more with respect to the total peak area of 100.
<8> The polythiol composition according to any one of <5> to <7>, wherein a ratio of the peak area of the component V with respect to the total peak area of 100 to the peak area of the component W with respect to the total peak area of 100 is from 0.10 to 2.80.
<9> The polythiol composition according to any one of <5> to <8>, further comprising at least one of a component X, which has a retention time of from 30.0 minutes to 40.0 minutes in the high-performance liquid chromatography measurement performed under the measurement conditions A, or a component Y, for which the retention time is from 60.0 minutes to 70.0 minutes.
<10> A polymerizable composition, comprising:
   the polythiol composition according to any one of <1> to <9>; and
   a polyiso(thio)cyanate compound.
<11> The polymerizable composition according to <10>, further comprising a thiol compound XS which is a thiol compound other than polythiol compounds contained in the polythiol composition.
<12> The polymerizable composition according to <11>, wherein the thiol compound XS is at least one selected from the group consisting of methanedithiol, 1,2-ethanedithiol, 1,2,3-propanetrithiol, pentaerythritol tetrakis(2-mercaptoacetate), pentaerythritol tetrakis(3-mercaptopropionate), tetrakis(mercaptomethylthiomethyl)methane, tetrakis(2-mercaptoethylthiomethyl)methane, tetrakis(3-mercaptopropylthiomethyl)methane, bis(2-mercaptoethyl) sulfide, bis(2,3-dimercaptopropyl) sulfide, 2,5-dimercaptomethyl-1,4-dithiane, 2,5-dimercapto-1,4-dithiane, 2,5-dimercaptomethyl-2,5-dimethyl-1,4-dithiane, 1,1,3,3-tetrakis(mercaptomethylthio)propane, 1,1,2,2-tetrakis(mercaptomethylthio)ethane, and 4,6-bis(mercaptomethylthio)-1,3-dithiane.
<13> The polymerizable composition according to any one of <10> to <12>, wherein the polyiso(thio)cyanate compound comprises at least one selected from the group consisting of pentamethylene diisocyanate, hexamethylene diisocyanate, xylylene diisocyanate, isophorone diisocyanate, bis(isocyanatomethyl)cyclohexane, bis(isocyanatocyclohexyl)methane, 2,5-bis(isocyanatomethyl)bicyclo-[2.2.1]-heptane, 2,6-bis(isocyanatomethyl)bicyclo-[2.2.1]-heptane, tolylene diisocyanate, 4,4'-diphenylmethane diisocyanate, and phenylene diisocyanate.
<14> The polymerizable composition according to any one of <10> to <13>, comprising a polyiso(thio)cyanate composition comprising the polyiso(thio)cyanate compound,
   wherein:
   the polyiso(thio)cyanate composition comprises:
      xylylene diisocyanate, and
      at least one selected from the group consisting of the following compounds (N1), (N2), and (N3);
   when the polyiso(thio)cyanate composition comprises the compound (N1), the compound (N1) has a peak area of 0.20 ppm or more with respect to a peak area of xylylene diisocyanate of 1 in a gas chromatography measurement;
   when the polyiso(thio)cyanate composition comprises the compound (N2), the compound (N2) has a peak area of 0.05 ppm or more with respect to a peak area of xylylene diisocyanate of 1 in a gas chromatography measurement; and
   when the polyiso(thio)cyanate composition comprises the compound (N3), the compound (N3) has a peak area of 0.10 ppm or more with respect to a peak area of xylylene diisocyanate of 1 in a gas chromatography measurement 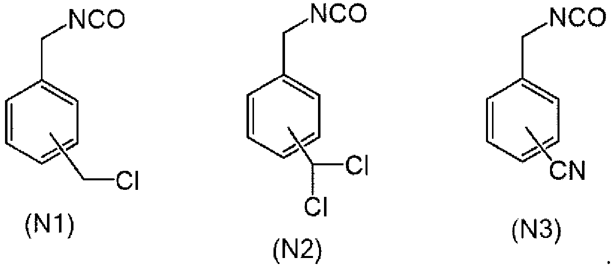
<15> A resin, comprising a cured product of the polymerizable composition according to any one of <10> to <14>.
<16> A molded body, comprising the resin according to <15>.
<17> An optical material, comprising the resin according to <15>.
<18> A lens, comprising the resin according to <15>.

### Advantageous Effects of Invention

According to the first and the second embodiments of the disclosure, the following can be provided: a polythiol composition from which a resin with reduced yellowness and improved heat resistance can be produced; and application thereof.

### DESCRIPTION OF EMBODIMENTS

In the disclosure, those numerical ranges that are expressed with "to" each denote a range that includes the numerical values stated before and after "to" as the minimum value and the maximum value, respectively.

In a set of numerical ranges that are stated in a stepwise manner in the disclosure, the upper limit value or the lower limit value of one numerical range may be replaced with the upper limit value or the lower limit value of other numerical range, or may be replaced with a relevant value indicated in any of Examples.

In the disclosure, when there are plural substances that correspond to a component of a material, an indicated amount of the component in the material means, unless otherwise specified, a total amount of the plural substances existing in the material.

The term "iso(thio)cyanate" used herein means isocyanate or isothiocyanate.

The first and the second embodiments of the disclosure will now be described.

There may be an overlapping part between the first and the second embodiments. For example, the polythiol composition of the first embodiment may have a characteristic feature of the polythiol composition of the second embodiment.

Hereinafter, with regard to a preferred characteristic feature in one of the embodiments, reference can be made as appropriate to a preferred characteristic feature of the other embodiment.

### [First Embodiment]

### <<Polythiol Composition>>

The polythiol composition of the first embodiment is a polythiol composition containing:
a polythiol component T which is at least one selected from the group consisting of 4,8-dimercaptomethyl-1,11-dimercapto-3,6,9-trithiaundecane (hereinafter, also referred to as "compound T1"), 4,7-dimercaptomethyl-1,11-dimercapto-3,6,9-trithiaundecane (hereinafter, also referred to as "compound T2"), and 5,7-dimercaptomethyl-1,11-dimercapto-3,6,9-trithiaundecane (hereinafter, also referred to as "compound T3");
a component X which has a retention time of from 30.0 minutes to 40.0 minutes in a high-performance liquid chromatography measurement performed under the following measurement conditions A; and
a component Y which has a retention time of from 60.0 minutes to 70.0 minutes.

In the high-performance liquid chromatography measurement, the polythiol component T has a peak area of 80.00 or more with respect to a total peak area of 100 of compounds contained in the polythiol composition, the component X has a peak area of 5.00 or less with respect to the total peak area of 100, and the component Y has a peak area of 2.50 or less with respect to the total peak area of 100.

### -Measurement Conditions A-

YMC-Pack (registered trademark) ODS-A manufactured by YMC Co., Ltd. (particle size S: 5 µm, column shape: Φ6 mm × 150 mm) is used as a column;
a mixed solution of acetonitrile/0.01-mol/L aqueous potassium dihydrogen phosphate solution = 60/40 (vol/vol) is used as a mobile phase;
a mixed solution of 160 mg of the polythiol composition and 10 mL of acetonitrile is used as a measurement solution;
a UV detector having a measurement wavelength of 230 nm is used as a detector;
a column temperature is set at 40°C;
a flow rate is set at 1.0 mL/min; and
an injection amount is set at 2 µL.

The above-described polythiol component T may be used as a polythiol component for forming a resin such as a plastic lens.

In this case, a reduction in yellowness and/or an improvement in heat resistance may be required for the resulting resin.

According to the polythiol composition of the first embodiment, a resin with reduced yellowness and improved heat resistance can be produced therefrom despite that the polythiol composition contains the polythiol component T.

The reason why such an effect is exerted is not clear; however, it is presumably because the amount of the component X and that of the component Y are reduced to the above-described respective amounts or less.

The polythiol composition of the first embodiment and preferred aspects thereof will now be described in more detail.

### <Polythiol Component T>

The polythiol composition of the first embodiment contains a polythiol component T.

The polythiol component T is at least one selected from the group consisting of 4,8-dimercaptomethyl-1,11-dimercapto-3,6,9-trithiaundecane (i.e., compound T1), 4,7-dimercaptomethyl-1,11-dimercapto-3,6,9-trithiaundecane (i.e., compound T2), and 5,7-dimercaptomethyl-1,11-dimercapto-3,6,9-trithiaundecane (i.e., compound T3).

The structures of the compounds T1 to T3 are as follows.

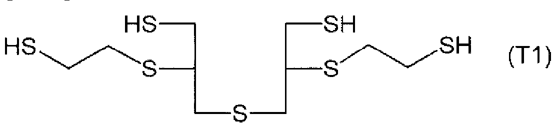

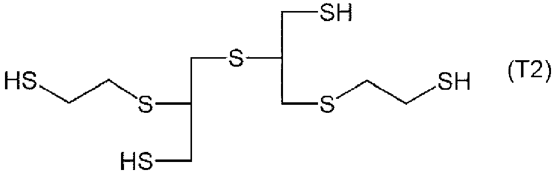

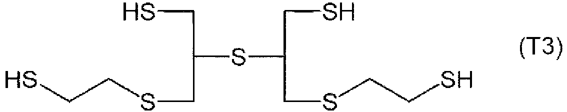

The polythiol component T does not necessarily have to be a mixture of the three compounds T1 to T3, and may be a mixture of two of the compounds T1 to T3, or may be any one of the compounds T1 to T3.

The three compounds T1 to T3 are in an isomeric relationship, and each has a molecular weight of 366.

When a total peak area of the polythiol component T, which is measured by high-performance liquid chromatography (hereinafter, also referred to as "HPLC") under the above-described measurement conditions A, is taken as 100, the compound T1 has a peak area of preferably 80 or more, more preferably from 80 to 90.

When the total peak area of the polythiol component T is taken as 100, the compound T2 has a peak area of preferably 20 or less, more preferably from 2 to 20, still more preferably from 5 to 20, yet still more preferably from 10 to 20.

When the total peak area of the polythiol component T is taken as 100, the compound T3 has a peak area of preferably 15 or less, more preferably 10 or less, still more preferably 5.0 or less, yet still more preferably 2.0 or less, further preferably 1.0 or less, still further preferably from 0.1 to 1.0.

The peak area of each of the compounds T1 to T3 and the peak area ratio of two or three of the compounds T1 to T3 can be adjusted by adjusting the production conditions (e.g., the reaction temperature and/or the reaction time in a thiuronium chlorination reaction using thiourea) for the production of a polythiol composition containing the polythiol component T (see, for example, Example 1 described below).

In HPLC under the above-described measurement conditions A, the polythiol component T has a peak area of 80.00 or more with respect to a total peak area of 100 of compounds contained in the polythiol composition.

In other words, the polythiol component T is a main component in the polythiol composition of the first embodiment.

The peak area of the polythiol component T is preferably from 80.00 to 90.00.

### <Component X>

The polythiol composition of the first embodiment contains a component X.

The component X is a component having a retention time of from 30.0 minutes to 40.0 minutes in HPLC under the above-described measurement conditions A.

The component X is not limited to being a single kind of compound, and may be two or more kinds of compounds (two or more kinds of compounds that are in an isomeric relationship).

In HPLC under the above-described measurement conditions A, the component X has a peak area of 5.00 or less.

By this, not only the yellowness is reduced but also the heat resistance is improved in the resulting resin.

The peak area of the component X is preferably 4.50 or less, more preferably 4.00 or less.

A lower limit of the peak area of the component X is not particularly limited, and may be, for example, 0.10, 0.50, or 1.00.

The component X is preferably a polythiol compound having a molecular weight of 426.

The molecular formula of the component X in this aspect is, for example, C₁₂H₂₆S₈.

The component X, which is a polythiol compound having a molecular weight of 426, is a compound having a molecular weight that is larger by 60 than that of each of the compounds T1 to T3 (molecular weight: 366).

The component X, which is a polythiol compound having a molecular weight of 426, is, for example, a compound in which at least one of the compounds T1 to T3 is substituted with a mercaptoethyl group (e.g., any of the compounds (X1) to (X3) below).

Examples of the component X, which is a polythiol compound having a molecular weight of 426, include the following compounds (X1), (X2), and (X3).

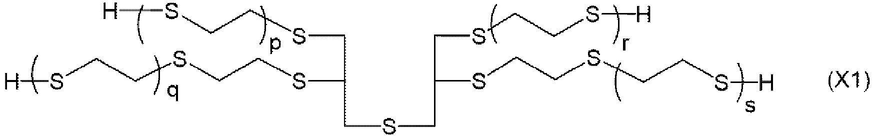

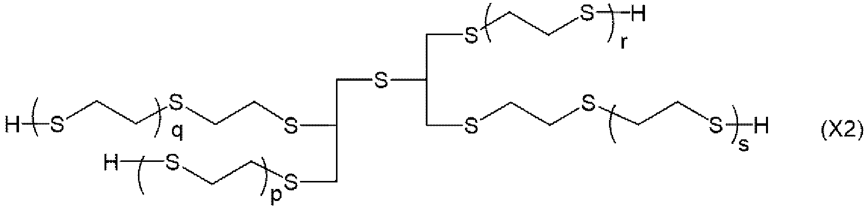

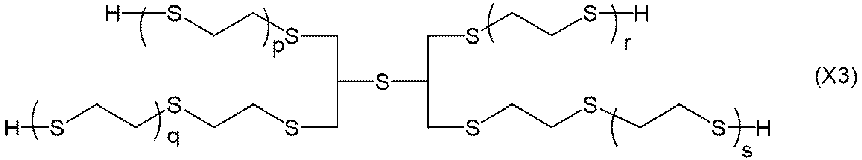

In the compounds (X1) to (X3), p, q, r, and s each represent 0 or 1, and a sum of p, q, r, and s is 1.

### <Component Y>

The polythiol composition of the first embodiment contains a component Y.

The component Y is a component having a retention time of from 60.0 minutes to 70.0 minutes in HPLC under the above-described measurement conditions A.

The component Y is not limited to being a single kind of compound, and may be two or more kinds of compounds (two or more kinds of compounds that are in an isomeric relationship).

In HPLC under the above-described measurement conditions A, the component Y has a peak area of 2.50 or less.

By this, not only the yellowness is reduced but also the heat resistance is improved in the resulting resin.

A lower limit of the peak area of the component Y is not particularly limited, and may be, for example, 0.10, 0.50, or 1.00.

The component Y is preferably a polythiol compound having a molecular weight of 532.

The molecular formula of the component Y in this aspect is, for example, C₁₅H₃₂S₁₀.

The component Y, which is a polythiol compound having a molecular weight of 532, is a compound having a molecular weight that is larger by 166 than that of each of the compounds T1 to T3 (molecular weight: 366).

Examples of the component Y, which is a polythiol compound having a molecular weight of 532, include the following compounds (Y1), (Y2), and (Y3).

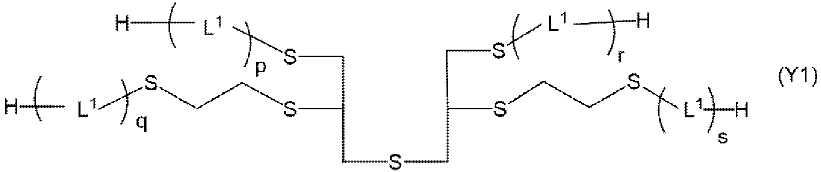

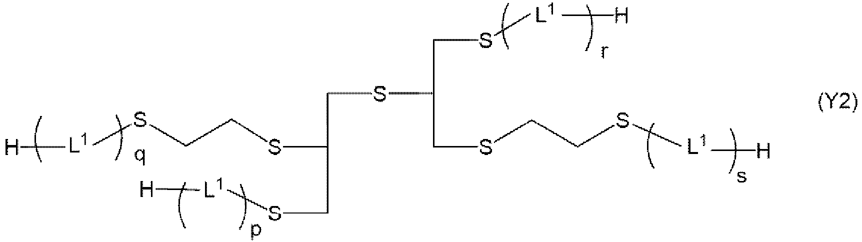

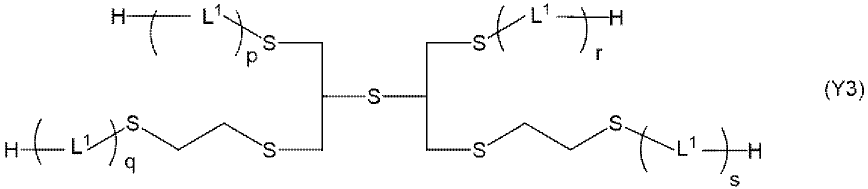

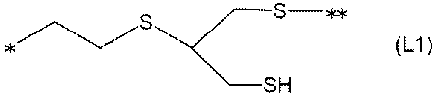

In the compounds (Y1) to (Y3), p, q, r, and s each represent 0 or 1; a sum of p, q, r, and s is 1; and L¹ represents a linking group (L1).

In the linking group (L1), * represents a binding position with a sulfur atom, and ** represents a binding position with a hydrogen atom.

### <Peak Area Ratio [Component Y/Component X]>

In the polythiol composition of the first embodiment, a ratio of the peak area of the component Y to the peak area of the component X (in the first embodiment, this ratio is also referred to as "peak area ratio [component Y/component X]") is not particularly limited; however, the peak area ratio [component Y/component X] is preferably from 0.20 to 3.00, more preferably from 0.30 to 2.00, still more preferably from 0.30 to 1.10, yet still more preferably from 0.30 to 1.00, further preferably from 0.40 to 1.00, still further preferably from 0.40 to 0.90, yet still further preferably from 0.55 to 0.80.

### <Other Components>

The polythiol composition of the first embodiment may also contain at least one other component (e.g., other polythiol compound, a component other than a polythiol compound) in addition to the above-described components.

Examples of the other polythiol compound include methanedithiol, 1,2-ethanedithiol, 1,2,3-propanetrithiol, pentaerythritol tetrakis(2-mercaptoacetate), pentaerythritol tetrakis(3-mercaptopropionate), bis(mercaptoethyl) sulfide, 2,5-dimercaptomethyl-1,4-dithiane, tetrakis(mercaptomethylthiomethyl)methane, tetrakis(2-mercaptoethylthiomethyl)methane, tetrakis(3-mercaptopropylthiomethyl)methane, bis(2,3-dimercaptopropyl) sulfide, 2,5-dimercaptomethyl-1,4-dithiane, 2,5-dimercapto-1,4-dithiane, 2,5-dimercaptomethyl-2,5-dimethyl-1,4-dithiane, 1,1,3,3-tetrakis(mercaptomethylthio)propane, 1,1,2,2-tetrakis(mercaptomethylthio)ethane, 4,6-bis(mercaptomethylthio)-1,3-dithiane, and 4-mercaptomethyl-1,8-dimercapto-3,6-dithiaoctane.

Examples of other components that may be contained in the polythiol composition of the first embodiment also include the components W and V and the compounds (XB) and (C1) in the below-described second embodiment.

### <Method of Producing Polythiol Composition>

The polythiol composition of the first embodiment can be produced by, for example, by adjusting the production conditions (e.g., the reaction temperature and/or the reaction time in a thiuronium chlorination reaction using thiourea) for the production of a polythiol composition containing the polythiol component T, and adjusting the constitution (particularly, the amounts of the components X and Y) of the polythiol composition (see, for example, Example 1 described below).

Alternatively, the polythiol composition of the first embodiment can also be produced by dissolving a polythiol composition containing the polythiol component T in an organic solvent (e.g., toluene), reacting the resulting solution with an alkaline substance (e.g., sodium hydroxide or potassium hydroxide) to form a salt of the polythiol composition (hereinafter, also referred to as "salt formation"), and then repeatedly performing extraction with water and separation to adjust the constitution (particularly, the amounts of the components X and Y) of the polythiol composition.

### <<Polymerizable Composition>>

The polymerizable composition of the first embodiment contains the polythiol composition of the first embodiment and a polyiso(thio)cyanate compound.

### (Polyiso(thio)cyanate Compound)

The polyiso(thio)cyanate compound is not particularly limited as long as the effects of the first embodiment can be exerted, and any conventionally known compound can be used. The polyiso(thio)cyanate compound is not particularly limited as long as it is a compound having at least two iso(thio)cyanate groups in one molecule, and specific examples thereof include:
aliphatic polyisocyanate compounds, such as tetramethylene diisocyanate, pentamethylene diisocyanate, hexamethylene diisocyanate, heptamethylene diisocyanate, octamethylene diisocyanate, 2,2,4-trimethylhexamethylene diisocyanate, 2,4,4-trimethylhexamethylene diisocyanate, lysine diisocyanate methyl ester, lysine triisocyanate, and xylylene diisocyanate;
alicyclic polyisocyanate compounds, such as isophorone diisocyanate, bis(isocyanatomethyl)cyclohexane, bis(isocyanatocyclohexyl)methane, dicyclohexyldimethylmethane diisocyanate, 2,5-bis(isocyanatomethyl)bicyclo-[2.2.1]-heptane, 2,6-bis(isocyanatomethyl)bicyclo-[2.2.1]-heptane, 3,8-bis(isocyanatomethyl)tricyclodecane, 3,9-bis(isocyanatomethyl)tricyclodecane, 4,8-bis(isocyanatomethyl)tricyclodecane, and 4,9-bis(isocyanatomethyl)tricyclodecane;
aromatic polyisocyanate compounds, such as tolylene diisocyanate, 4,4'-diphenylmethane diisocyanate, diphenyl sulfide-4,4'-diisocyanate, and phenylene diisocyanate;
heterocyclic polyisocyanate compounds, such as 2,5-diisocyanatothiophene, 2,5-bis(isocyanatomethyl)thiophene, 2,5-diisocyanatotetrahydrothiophene, 2,5-bis(isocyanatomethyl)tetrahydrothiophene, 3,4-bis(isocyanatomethyl)tetrahydrothiophene, 2,5-diisocyanato-1,4-dithiane, 2,5-bis(isocyanatomethyl)-1,4-dithiane, 4,5-diisocyanato-1,3-dithiolane, and 4,5-bis(isocyanatomethyl)-1,3-dithiolane;
aliphatic polyisothiocyanate compounds, such as hexamethylene diisothiocyanate, lysine diisothiocyanate methyl ester, lysine triisothiocyanate, and xylylene diisothiocyanate;
alicyclic polyisothiocyanate compounds, such as isophorone diisothiocyanate, bis(isothiocyanatomethyl)cyclohexane, bis(isothiocyanatocyclohexyl)methane, cyclohexane diisothiocyanate, methylcyclohexane diisothiocyanate, 2,5-bis(isothiocyanatomethyl)bicyclo-[2.2.1]-heptane, 2,6-bis(isothiocyanatomethyl)bicyclo-[2.2.1]-heptane, 3,8-bis(isothiocyanatomethyl)tricyclodecane, 3,9-bis(isothiocyanatomethyl)tricyclodecane, 4,8-bis(isothiocyanatomethyl)tricyclodecane, and 4,9-bis(isothiocyanatomethyl)tricyclodecane;
aromatic polyisothiocyanate compounds, such as tolylene diisothiocyanate, 4,4'-diphenylmethane diisothiocyanate, and diphenyl disulfide-4,4'-diisothiocyanate; and
sulfur-containing heterocyclic polyisothiocyanate compounds, such as 2,5-diisothiocyanatothiophene, 2,5-bis(isothiocyanatomethyl)thiophene, 2,5-isothiocyanatotetrahydrothiophene, 2,5-bis(isothiocyanatomethyl)tetrahydrothiophene, 3,4-bis(isothiocyanatomethyl)tetrahydrothiophene, 2,5-diisothiocyanato-1,4-dithiane, 2,5-bis(isothiocyanatomethyl)-1,4-dithiane, 4,5-diisothiocyanato-1,3-dithiolane, and 4,5-bis(isothiocyanatomethyl)-1,3-dithiolane.

The polyiso(thio)cyanate compound may contain at least one selected from the above-exemplified compounds.

As the polyiso(thio)cyanate compound, for example, a halogen substitute such as a chlorine substitute or a bromine substitute, an alkyl substitute, an alkoxy substitute, a nitro substitute, a prepolymer-type product modified with a polyhydric alcohol, a carbodiimide-modified product, a urea-modified product, a burette-modified product, or a dimerization or trimerization reaction product, of any of the above-exemplified compounds can be used as well.

The polyiso(thio)cyanate compound is preferably a polyisocyanate compound, and more preferably contains at least one selected from the group consisting of pentamethylene diisocyanate, hexamethylene diisocyanate, xylylene diisocyanate, isophorone diisocyanate, bis(isocyanatomethyl)cyclohexane, bis(isocyanatocyclohexyl)methane, 2,5-bis(isocyanatomethyl)bicyclo-[2.2.1]-heptane, 2,6-bis(isocyanatomethyl)bicyclo-[2.2.1]-heptane, tolylene diisocyanate, 4,4'-diphenylmethane diisocyanate, and phenylene diisocyanate.

A mixing ratio of the polythiol composition and the polyiso(thio)cyanate compound is not particularly limited and, for example, a molar ratio between the mercapto groups of the polythiol compound contained in the polythiol composition and the iso(thio)cyanate groups of the polyiso(thio)cyanate compound (mercapto groups/iso(thio)cyanate groups) is preferably from 0.5 to 3.0, more preferably from 0.6 to 2.0, still more preferably from 0.8 to 1.3. When the mixing ratio is in the above-described range, various performance required for a plastic lens or the like, such as refractive index and heat resistance, tend to be satisfied in a well-balanced manner.

The polymerizable composition of the first embodiment may contain a polyiso(thio)cyanate composition containing the above-described polyiso(thio)cyanate compound.

The term "polyisocyanate composition" used herein means a composition containing at least one polyisocyanate compound.

The polyisocyanate composition may also contain, as an impurity, a component other than the polyisocyanate compound.

It is preferred that the polyisocyanate composition contains at least one polyisocyanate compound as a main component.

The meaning of "contain ... as a main component" is as described above.

The polyisocyanate composition preferably contains xylylene diisocyanate.

Hereinafter, a polyisocyanate composition containing xylylene diisocyanate is also referred to as "XDI composition".

The XDI composition preferably contains xylylene diisocyanate as a main component.

The XDI composition preferably contains at least one selected from the group consisting of the following compounds (N1), (N2), and (N3).

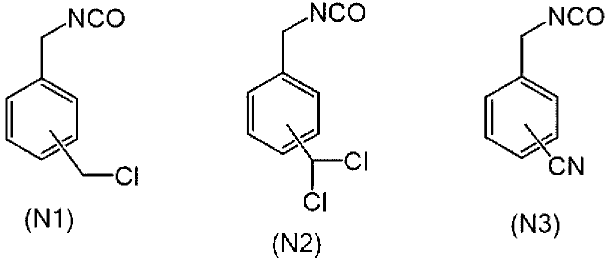

Aspects of the XDI composition that are preferred from standpoint of obtaining superior stability of the polyiso(thio)cyanate composition and superior transparency of a resin formed using the polyiso(thio)cyanate composition will now be described.

When the XDI composition contains the compound (N1), the compound (N1) preferably has a peak area of 0.20 ppm or more with respect to a peak area of xylylene diisocyanate of 1 in a gas chromatography measurement performed under the following GC conditions 1:
- GC Conditions 1-
   Filler: DB-1 (film thickness) 1.5 µm
   Column: 0.53 mm in inner diameter × 60 m in length (manufactured by Agilent Technologies, Inc.)
   Oven temperature: increased from 130°C to 220°C at 3°C/min, and further increased to 300°C at 10°C/min after reaching 220°C
   Split ratio: pulsed splitless method
   Inlet temperature: 280°C
   Detector temperature: 300°C
   Carrier gas: N₂ 158 kPa, H₂ 55 kPa, Air 45 kPa (constant pressure control)
   Solvent: chloroform
   Sample concentration: 2.0%-by-mass chloroform solution
   Injection amount: 2 µL
   Detection method: FID

The peak area of the compound (N1) is more preferably 5.0 ppm or more, still more preferably 50 ppm or more, yet still more preferably 100 ppm or more, with respect to a peak area of xylylene diisocyanate of 1.

The peak area of the compound (N1) is preferably 4,000 ppm or less, more preferably 3,000 ppm or less, still more preferably 2,000 ppm or less, yet still more preferably 1,500 ppm or less, further preferably 1,000 ppm or less, with respect to a peak area of xylylene diisocyanate of 1.

The peak area of the compound (N1) can be measured in accordance with the method described in the paragraph [0377] of Japanese Patent No. 6373536.

When the XDI composition contains the compound (N2), the compound (N2) preferably has a peak area of 0.05 ppm or more with respect to a peak area of xylylene diisocyanate of 1 in a gas chromatography measurement performed under the following GC conditions 2:
- GC Conditions 2-
   Column: HP-50+, 0.25 mm in inner diameter × 30 m in length × 0.25 µm in film thickness (manufactured by Hewlett-Packard Company)
   Oven temperature: increased from 50°C to 280°C at 10°C/min, and held for 6 minutes after reaching 280°C
   Split ratio: pulsed splitless method
   Inlet temperature: 200°C
   Detector temperature: 280°C
   Carrier gas: He
   Carrier gas flow rate: 1.0 ml/min (constant flow rate control)
   Sample concentration: 1.0%-by-mass dichloromethane solution
   Injection amount: 1.0 µL
   Detection method: SIM (monitoring ion: m/z 180, 215) (content ratio of xylylene diisocyanate (XDI)).

The peak area of the compound (N2) is more preferably 0.1 ppm or more, still more preferably 0.3 ppm or more, yet still more preferably 0.6 ppm or more, with respect to a peak area of xylylene diisocyanate of 1.

The peak area of the compound (N2) is preferably 200 ppm or less, more preferably 150 ppm or less, still more preferably 100 ppm or less, yet still more preferably 80 ppm or less, further preferably 70 ppm or less, yet further preferably 60 ppm or less, with respect to a peak area of xylylene diisocyanate of 1.

The peak area of the compound (N2) can be measured in accordance with the method described in the paragraphs [0375] and [0376] of Japanese Patent No. 6373536.

When the XDI composition contains the compound (N3), the compound (N3) preferably has a peak area of 0.10 ppm or more with respect to a peak area of xylylene diisocyanate of 1 in a gas chromatography measurement performed under the above-described GC conditions 1.

The peak area of the compound (N3) is more preferably 0.1 ppm or more, still more preferably 3.0 ppm or more, yet still more preferably 5.0 ppm or more, with respect to a peak area of xylylene diisocyanate of 1.

The peak area of the compound (N3) is preferably 1,000 ppm or less, more preferably 500 ppm or less, still more preferably 300 ppm or less, yet still more preferably 100 ppm or less, further preferably 75 ppm or less, with respect to a peak area of xylylene diisocyanate of 1.

The peak area of the compound (N3) can be measured in accordance with the method described in the paragraph [0377] of Japanese Patent No 6373536.

The XDI composition has an acid content of preferably 3,000 ppm or less, more preferably 2,000 ppm or less, still more preferably 1,000 ppm or less, yet still more preferably 100 ppm or less, further preferably 50 ppm or less, yet further preferably 30 ppm or less, further more preferably less than 15 ppm.

A lower limit value of the acid content of the XDI composition is not particularly limited; however, it is, for example, 1 ppm.

The acid content of the XDI composition can be measured in accordance with the method described in the paragraph [0091] of WO 2021/256417.

The XDI composition may also contain a stabilizer.

The polymerizable composition of the first embodiment may further contain a thiol compound XS, which is exemplified in the below-described second embodiment.

The polymerizable composition of the first embodiment may also contain other components in addition to the polythiol compound and the polyiso(thio)cyanate compound, for the purpose of improving various physical properties of the resin, operability, polymerization reactivity of the polymerizable composition, and the like.

Examples of the other components include a polymerization catalyst, an internal mold release agent, a resin modifier, a chain extender, a crosslinking agent, a radical scavenger, a light stabilizer, a UV absorber, an antioxidant, an oil-soluble dye, a filler, an adhesion improver, an antimicrobial agent, an antistatic agent, a dye, a fluorescent brightener, a fluorescent pigment, and a blue ink agent such as an inorganic pigment.

Examples of the polymerization catalyst include tertiary amine compounds, inorganic and organic acid salts thereof, metal compounds, quaternary ammonium salts, and organic sulfonic acids.

As the internal mold release agent, an acidic phosphoric acid ester can be used. Examples of the acidic phosphoric acid ester include phosphoric acid monoesters and phosphoric acid diesters, and these may be used singly, or in combination of two or more kinds thereof.

Examples of the resin modifier include episulfide compounds, alcohol compounds, amine compounds, epoxy compounds, organic acids and anhydrides thereof, and olefin compounds containing a (meth)acrylate compound or the like.

The polymerizable composition of the first embodiment can be obtained by mixing the above-described components.

### <<Molded Body>>

The molded body of the first embodiment includes the resin of the first embodiment.

The resin of the first embodiment includes a cured product of the polymerizable composition of the first embodiment.

A method of producing the molded body of the first embodiment is not particularly limited, and one example of a preferred production method is cast polymerization. First, the polymerizable composition is injected between casting molds held by a gasket, a tape, or the like. In this process, depending on the physical properties required for the plastic lens to be obtained, it is often preferred to perform, for example, a defoaming treatment under reduced pressure, and a filtration treatment under pressure, reduced pressure, or the like, if necessary.

The polymerization conditions are not limited since they are greatly variable depending on the constitution of the polymerizable composition, the type and the amount of a catalyst to be used, the shape of the molds, and the like, and the polymerization is performed, for example, at a temperature of from -50°C to 150°C over a period of from 1 hour to 50 hours. In some cases, it is preferred to maintain or gradually increase the temperature in a range of from 10°C to 150°C and thereby cure the polymerizable composition for 1 hour to 48 hours.

If necessary, the molded body may be subjected to a treatment such as annealing. The treatment such as annealing is performed in a range of usually from 50°C to 150°C, preferably from 90°C to 140°C, more preferably from 100°C to 130°C.

### [Application]

The resin obtained from the polymerizable composition of the first embodiment can be used as a material for producing molded bodies of various shapes by changing the type of the molds used for cast polymerization.

### <<Optical Material>>

The optical material of the first embodiment contains the resin of the first embodiment.

A molded body obtained from the polymerizable composition of the first embodiment can provide a material in which the yellowness is reduced without impairing the transparency. Further, a molded body obtained from a polymerizable composition containing the polythiol composition of the first embodiment can provide a material that has excellent degree of devitrification as well.

Therefore, the polythiol composition of the first embodiment can be used for various optical materials such as plastic lenses.

### <<Lens>>

The lens of the first embodiment contains the resin of the first embodiment.

As the optical material, a lens is particularly suitable.

Examples of the lens include plastic eyeglass lenses and plastic polarizing lenses.

### [Plastic Eyeglass Lens]

A plastic eyeglass lens using a lens substrate made of the molded body of the first embodiment may have a coating layer applied to one or both sides, if necessary.

The plastic eyeglass lens of the first embodiment includes a lens substrate containing a cured product of the above-described polymerizable composition, and a coating layer.

Specific examples of the coating layer include a primer layer, a hard coat layer, an anti-reflection layer, an anti-fog coating layer, an anti-fouling layer, and a water-repellent layer. These coating layers may each be used singly, or plural coating layers may be used in the form of a multilayer structure. When coating layers are formed on both sides, the same coating layer may be formed on the respective sides, or different coating layers may be formed on the respective sides.

These coating layers may each be used in combination with known additives, such as an infrared absorber for protecting the eyes from infrared rays; a light stabilizer, an antioxidant, or the like for improving the weather resistance of the lens; a photochromic compound, a dye, a pigment, or the like for improving the fashionability of the lens; and an antistatic agent for enhancing the performance of the lens.

With regard to layers to be coated by application, various leveling agents for improving the coating properties may be used.

Further, on an anti-reflection layer, if necessary, an anti-fogging layer, an anti-fouling layer, or a water-repellent layer may be formed.

The first embodiment has been described thus far; however, the above-described constitutions are merely examples, and various other constitutions can be adopted within a range that does not impair the effects of the first embodiment.

### [Second Embodiment]

### <<Polythiol Composition>>

The polythiol composition of the second embodiment is a polythiol composition containing:
a polythiol component T which is at least one selected from the group consisting of 4,8-dimercaptomethyl-1,11-dimercapto-3,6,9-trithiaundecane (hereinafter, also referred to as "compound T1"), 4,7-dimercaptomethyl-1,11-dimercapto-3,6,9-trithiaundecane (hereinafter, also referred to as "compound T2"), and 5,7-dimercaptomethyl-1,11-dimercapto-3,6,9-trithiaundecane (hereinafter, also referred to as "compound T3");
a component W which has a retention time of from 8.7 minutes to 9.7 minutes in a high-performance liquid chromatography measurement performed under the following measurement conditions A; and
a component V which has a retention time of from 11.0 minutes to 13.5 minutes.

In the high-performance liquid chromatography measurement, the polythiol component T has a peak area of 75.00 or more with respect to a total peak area of 100 of compounds contained in the polythiol composition, the component W has a peak area of 3.50 or less with respect to the total peak area of 100, and the component V has a peak area of 3.10 or less with respect to the total peak area of 100.

### -Measurement Conditions A-

YMC-Pack (registered trademark) ODS-A manufactured by YMC Co., Ltd. (particle size S: 5 µm, column shape: Φ6 mm × 150 mm) is used as a column;
a mixed solution of acetonitrile/0.01-mol/L aqueous potassium dihydrogen phosphate solution = 60/40 (vol/vol) is used as a mobile phase;
a mixed solution of 160 mg of the polythiol composition and 10 mL of acetonitrile is used as a measurement solution;
a UV detector having a measurement wavelength of 230 nm is used as a detector;
a column temperature is set at 40°C;
a flow rate is set at 1.0 mL/min; and
an injection amount is set at 2 µL.

The measurement conditions A in the second embodiment are the same as the measurement conditions A in the above-described first embodiment.

The above-described polythiol component T may be used as a polythiol component for forming a resin such a plastic lens.

In this case, a reduction in yellowness and/or an improvement in heat resistance may be required for the resulting resin.

According to the polythiol composition of the second embodiment, a resin with reduced yellowness and improved heat resistance can be produced therefrom despite that the polythiol composition contains the polythiol component T.

The reason why such an effect is exerted is not clear; however, it is presumably because the amount of the component W and that of the component V are reduced to the above-described respective amounts or less.

The polythiol composition of the second embodiment and preferred aspects thereof will now be described in more detail.

### <Polythiol Component T>

The polythiol composition of the second embodiment contains a polythiol component T.

The polythiol component T in the second embodiment is the same as the polythiol component T in the above-described first embodiment.

In other words, the polythiol component T is at least one selected from the group consisting of 4,8-dimercaptomethyl-1,11-dimercapto-3,6,9-trithiaundecane (i.e., compound T1), 4,7-dimercaptomethyl-1,11-dimercapto-3,6,9-trithiaundecane (i.e., compound T2), and 5,7-dimercaptomethyl-1,11-dimercapto-3,6,9-trithiaundecane (i.e., compound T3).

The structures of the compounds T1 to T3 are as follows.

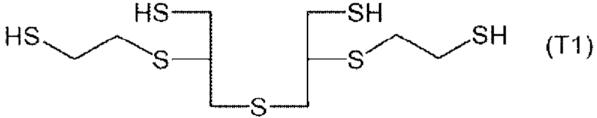

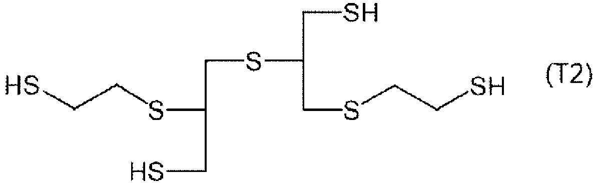

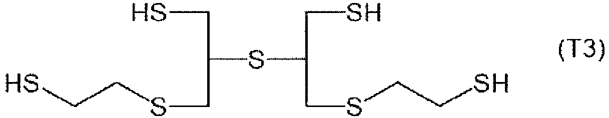

The polythiol component T does not necessarily have to be a mixture of the three compounds T1 to T3, and may be a mixture of two of the compounds T1 to T3, or may be any one of the compounds T1 to T3.

The three compounds T1 to T3 are in an isomeric relationship, and each has a molecular weight of 366.

When a total peak area of the polythiol component T, which is measured by high-performance liquid chromatography (hereinafter, also referred to as "HPLC") under the above-described measurement conditions A, is taken as 100, the compound T1 has a peak area of preferably 77.00 or more, more preferably 80.00 or more, still more preferably 82.00 or more, yet still more preferably 84.00 or more.

An upper limit of the peak area of the compound T1 is preferably 90.00.

When the total peak area of the polythiol component T is taken as 100, the compound T2 has a peak area of preferably 21.00 or less, more preferably 19.00 or less, still more preferably 17.00 or less.

A lower limit of the peak area of the compound T2 is preferably 10.00.

When the total peak area of the polythiol component T is taken as 100, the compound T3 has a peak area of preferably 3.00 or less, more preferably 1.50 or less, still more preferably 0.70 or less.

The peak area of the compound T3 may be 0.00.

The peak area of each of the compounds T1 to T3 and the peak area ratio of two or three of the compounds T1 to T3 can be adjusted by adjusting the production conditions (e.g., the reaction temperature and/or the reaction time in a thiuronium chlorination reaction using thiourea) for the production of a polythiol composition containing the polythiol component T (see, for example, Example 101 described below).

In HPLC under the above-described measurement conditions A, the peak area of the polythiol component T with respect to a total peak area of 100 of compounds contained in the polythiol composition (hereinafter, also simply referred to as "peak area of the polythiol component T") is 75.00 or more.

In other words, the polythiol component T is a main component in the polythiol composition of the second embodiment.

The peak area of the polythiol component T is preferably 80.00 or more, more preferably 84.00 or more, still more preferably 85.00 or more.

The peak area of the polythiol component T may be 100.

### <Component W>

The polythiol composition of the second embodiment contains a component W.

The component W is a component having a retention time of from 8.7 minutes to 9.7 minutes in HPLC under the above-described measurement conditions A.

The component W is not limited to being a single kind of compound, and may be two or more kinds of compounds (two or more kinds of compounds that are in an isomeric relationship).

In HPLC under the above-described measurement conditions A, the peak area of the component W with respect to a total peak area of 100 of compounds contained in the polythiol composition (hereinafter, also simply referred to as "peak area of the component W") is 3.50 or less.

By this, not only the yellowness is reduced but also the heat resistance is improved in the resulting resin.

The peak area of the component W is preferably 3.00 or less, more preferably 2.75 or less.

A lower limit of the peak area of the component W is preferably 1.20, more preferably 1.30, still more preferably 1.50, yet still more preferably 1.80.

The lower limit of the peak area of the component W is not particularly limited, and may be, for example, 1.20, 1.30, 1.50, or 1.80.

The component W is preferably a polythiol compound having a molecular weight of 350.

The molecular formula of the component W in this aspect is, for example, C₁₀H₂₂OS₆.

The component W, which is a polythiol compound having a molecular weight of 350, is a compound having a molecular weight that is smaller by 16 than that of each of the compounds T1 to T3 (molecular weight: 366).

The component W, which is a polythiol compound having a molecular weight of 350, is, for example, a compound in which one of the SH groups in at least one of the compounds T1 to T3 is substituted with an OH group (e.g., any of the compounds (W1) to (W5) below).

Examples of the component W, which is a polythiol compound having a molecular weight of 350, include the following compounds (W1) to (W5).

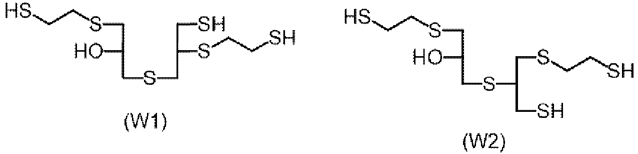

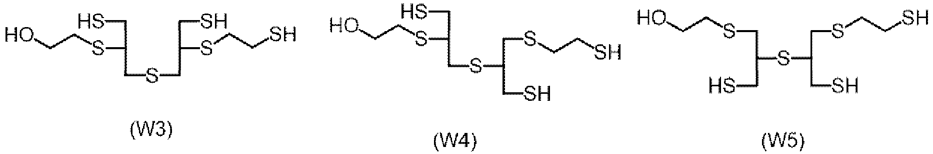

### <Component V>

The polythiol composition of the second embodiment contains a component V.

The component V is a component having a retention time of from 11.0 minutes to 13.5 minutes in HPLC under the above-described measurement conditions A.

The component V is not limited to being a single kind of compound, and may be two or more kinds of compounds (two or more kinds of compounds that are in an isomeric relationship).

In HPLC under the above-described measurement conditions A, the peak area of the component V with respect to a total peak area of 100 of compounds contained in the polythiol composition (hereinafter, also simply referred to as "peak area of the component V") is 3.10 or less.

By this, not only the yellowness is reduced but also the heat resistance is improved in the resulting resin.

The peak area of the component V is preferably 3.00 or less, more preferably 2.90 or less.

A lower limit of the peak area of the component V is not particularly limited, and may be, for example, 1.00, 1.30, or 1.60.

The component V is preferably a polythiol compound having a molecular weight of 260.

The molecular formula of the component V in this aspect is, for example, C₇H₁₆S₅.

Examples of the component V, which is a polythiol compound having a molecular weight of 260, include the following compound (V1).

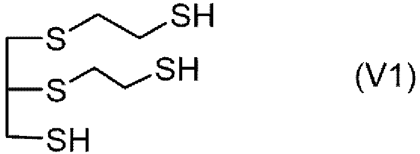

A preferred combination of the component W and the component V is a combination in which the component W is a polythiol compound having a molecular weight of 350, and the component V is a polythiol compound having a molecular weight of 260.

A preferred combination of the peak area of the component W and the peak area of the component V is a combination in which the peak area of the component W is 1.30 or more, and the peak area of the component V is 1.00 or more.

### <Peak Area Ratio [Component V/Component W]>

In the polythiol composition of the second embodiment, a ratio of the peak area of the component V (i.e., the peak area of the component V with respect to a total peak area of 100 of compounds contained in the polythiol composition) to the peak area of the component W (i.e., the peak area of the component W with respect to a total peak area of 100 of compounds contained in the polythiol composition) (in the second embodiment, this ratio is also referred to as "peak area ratio [component V/component W]") is preferably 2.80 or less, more preferably 2.40 or less, still more preferably 2.00 or less, yet still more preferably 1.50 or less.

A lower limit of the peak area ratio [component V/component W] is not particularly limited, and may be, for example, 0.10 or 0.50.

A preferred range of the peak area ratio [component V/component W] may be, for example, from 0.10 to 2.80.

### <Peak Area Ratio [Component V/Polythiol Component T]>

In the polythiol composition of the second embodiment, a ratio of the peak area of the component V to the peak area of the polythiol component T (in the second embodiment, this ratio is also referred to as "peak area ratio [component V/polythiol component T]") is preferably 0.06 or less, more preferably 0.03 or less.

A lower limit of the peak area ratio [component V/polythiol component T] is not particularly limited, and may be, for example, 0.01.

### <Component U>

The polythiol composition of the second embodiment may contain a component U.

The component U is a component having a retention time of from 40.0 minutes to 49.0 minutes in HPLC under the above-described measurement conditions A.

The component U is not limited to being a single kind of compound, and may be two or more kinds of compounds (two or more kinds of compounds that are in an isomeric relationship).

In HPLC under the above-described measurement conditions A, the peak area of the component U with respect to a total peak area of 100 of compounds contained in the polythiol composition (hereinafter, also simply referred to as "peak area of the component U") is preferably 1.70 or more, more preferably 2.10 or more, still more preferably 2.50 or more.

An upper limit of the peak area of the component U is not particularly limited, and may be, for example, 5.00 or 4.00.

The component U is preferably a polythiol compound having a molecular weight of 472.

The molecular formula of the component U in this aspect is, for example, C₁₃H₂₈S₉.

Examples of the component U, which is a polythiol compound having a molecular weight of 472, include the following compounds (U1) to (U4).

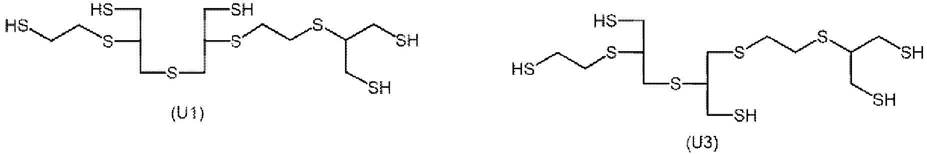

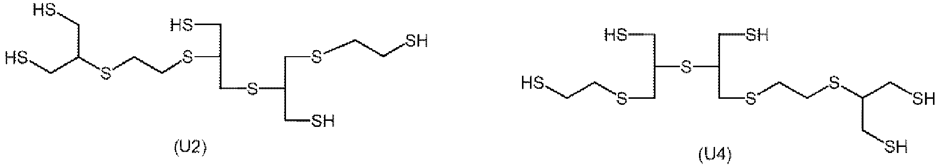

### <Component X and Component Y>

The polythiol composition of the second embodiment may further contain at least one of a component X which has a retention time of from 30.0 minutes to 40.0 minutes in a high-performance liquid chromatography measurement performed under the above-described measurement conditions A or a component Y which has a retention time of from 60.0 minutes to 70.0 minutes.

### (Component X)

The polythiol composition of the second embodiment may contain a component X.

The component X is a component having a retention time of from 30.0 minutes to 40.0 minutes in HPLC under the above-described measurement conditions A.

The component X is not limited to being a single kind of compound, and may be two or more kinds of compounds (two or more kinds of compounds that are in an isomeric relationship).

In HPLC under the above-described measurement conditions A, the peak area of the component X with respect to a total peak area of 100 of compounds contained in the polythiol composition (hereinafter, also simply referred to as "peak area of the component X") is preferably 6.00 or less.

By this, not only the yellowness is reduced but also the heat resistance is improved in the resulting resin.

The peak area of the component X is more preferably 5.00 or less, still more preferably 3.00 or less.

A lower limit of the peak area of the component X is not particularly limited, and may be, for example, 0.10 or 0.50.

The component X that may be contained in the polythiol composition of the second embodiment and preferred aspects thereof are the same as the component X contained in the above-described polythiol composition of the first embodiment and preferred aspects thereof.

### (Component Y)

The polythiol composition of the second embodiment may contain a component Y.

The component Y is a component having a retention time of from 60.0 minutes to 70.0 minutes in HPLC under the above-described measurement conditions A.

The component Y is not limited to being a single kind of compound, and may be two or more kinds of compounds (two or more kinds of compounds that are in an isomeric relationship).

In HPLC under the above-described measurement conditions A, the peak area of the component Y with respect to a total peak area of 100 of compounds contained in the polythiol composition (hereinafter, also simply referred to as "peak area of the component Y") is preferably 3.00 or less.

By this, not only the yellowness is reduced but also the heat resistance is improved in the resulting resin.

The peak area of the component Y is more preferably 2.60 or less, still more preferably 2.00 or less.

A lower limit of the peak area of the component Y is not particularly limited, and may be, for example, 0.10 or 0.50.

The component Y that may be contained in the polythiol composition of the second embodiment and preferred aspects thereof are the same as the component Y contained in the above-described polythiol composition of the first embodiment and preferred aspects thereof.

### (Peak Area Ratio [Component Y/Component X])

In the polythiol composition of the second embodiment, a ratio of the peak area of the component Y (i.e., the peak area of the component Y with respect to a total peak area of 100 of compounds contained in the polythiol composition) to the peak area of the component X (i.e., the peak area of the component X with respect to a total peak area of 100 of compounds contained in the polythiol composition) (in the second embodiment, this ratio is also referred to as "peak area ratio [component Y/component X]") is not particularly limited; however, the peak area ratio [component Y/component X] is preferably 0.40 or more, more preferably 0.60 or more, still more preferably 0.65 or more.

An upper limit of the peak area ratio [component Y/component X] is not particularly limited, and may be, for example, 1.50 or 1.20.

### <Compound (XB)>

The polythiol composition of the second embodiment may also contain the following compound (XB).

The compound (XB) is a compound in which at least one of the mercapto groups in the above-described polythiol component T or component V is substituted with a group represented by the following Formula (XB-1).

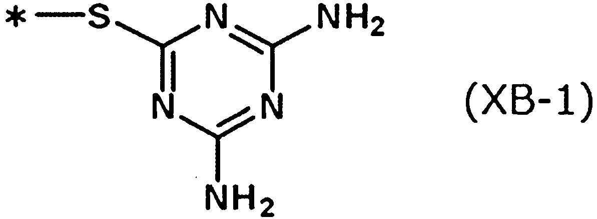

In Formula (XB-1), * represents a binding position.

Examples of the compound (XB) are provided below; however, the compound (XB) is not limited to the following examples.

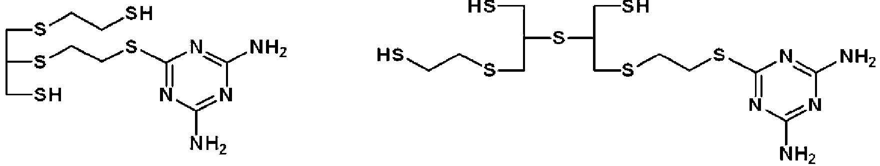

### <Compound (C1)>

The polythiol composition of the second embodiment may also contain the following compound (C1).

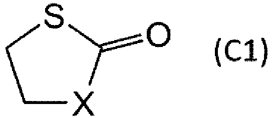

In the compound (C1), X represents -CH₂- or a sulfur atom.

### <Other Components>

The polythiol composition of the second embodiment may also contain at least one other component (e.g., other thiol compound, a component other than a thiol compound) in addition to the above-described components.

One example of the other thiol compound is the below-described thiol compound XS that may be contained in the polymerizable composition.

### <Method of Producing Polythiol Composition>

The polythiol composition of the second embodiment can be produced by, for example, by adjusting the production conditions (e.g., the reaction temperature and/or the reaction time in a thiuronium chlorination reaction using thiourea) for the production of a polythiol composition containing the polythiol component T, and adjusting the constitution (particularly, the amounts of the components W and V) of the polythiol composition (see, for example, Example 101 described below).

Alternatively, the polythiol composition of the second embodiment can be produced by dissolving a polythiol composition containing the polythiol component T in an organic solvent (e.g., toluene), reacting the resulting solution with an alkaline substance (e.g., sodium hydroxide or potassium hydroxide) to form a salt of the polythiol composition (hereinafter, also referred to as "salt formation"), and then repeatedly performing extraction with water and separation to adjust the constitution (particularly, the amounts of the components W and V) of the polythiol composition.

### <<Polymerizable Composition>>

The polymerizable composition of the second embodiment contains the polythiol composition of the second embodiment and a polyiso(thio)cyanate compound.

### <Polyiso(thio)cyanate Compound>

The polyiso(thio)cyanate compound is not particularly limited as long as the effects of the second embodiment can be exerted, and any conventionally known compound can be used. The polyiso(thio)cyanate compound is not particularly limited as long as it is a compound having at least two iso(thio)cyanate groups in one molecule.

Specific examples of the polyiso(thio)cyanate compound in the second embodiment are the same as those in the above-described first embodiment.

The polyiso(thio)cyanate compound is preferably a polyisocyanate compound, and more preferably contains at least one selected from the group consisting of pentamethylene diisocyanate, hexamethylene diisocyanate, xylylene diisocyanate, isophorone diisocyanate, bis(isocyanatomethyl)cyclohexane, bis(isocyanatocyclohexyl)methane, 2,5-bis(isocyanatomethyl)bicyclo-[2.2.1]-heptane, 2,6-bis(isocyanatomethyl)bicyclo-[2.2.1]-heptane, tolylene diisocyanate, 4,4'-diphenylmethane diisocyanate, and phenylene diisocyanate.

A mixing ratio of the polythiol composition and the polyiso(thio)cyanate compound is not particularly limited and, for example, a molar ratio between the mercapto groups of the polythiol compound contained in the polythiol composition and the iso(thio)cyanate groups of the polyiso(thio)cyanate compound (mercapto groups/iso(thio)cyanate groups) is preferably from 0.5 to 3.0, more preferably from 0.6 to 2.0, still more preferably from 0.8 to 1.3. When the mixing ratio is in the above-described range, various performance required for a plastic lens or the like, such as refractive index and heat resistance, tend to be satisfied in a well-balanced manner.

The polymerizable composition of the second embodiment may contain a polyiso(thio)cyanate composition containing the above-described polyiso(thio)cyanate compound.

The term "polyisocyanate composition" used herein means a composition containing at least one polyisocyanate compound.

The polyisocyanate composition may also contain, as an impurity, a component other than the polyisocyanate compound.

It is preferred that the polyisocyanate composition contains at least one polyisocyanate compound as a main component.

The meaning of "contain ... as a main component" is as described above.

The polyisocyanate composition preferably contains xylylene diisocyanate.

Hereinafter, a polyisocyanate composition containing xylylene diisocyanate is also referred to as "XDI composition".

The XDI composition preferably contains xylylene diisocyanate as a main component.

Preferred aspects of the XDI composition in the second embodiment are the same as those in the first embodiment.

For example, the XDI composition in the second embodiment preferably contains at least one selected from the group consisting of the above-described compounds (N1), (N2), and (N3), in the same manner as the XDI composition in the first embodiment. Preferred ranges of the peak areas determined by gas chromatography measurement for each of the compounds (N1), (N2), and (N3) in the second embodiment are also the same as the preferred ranges of the peak areas determined by gas chromatography measurement for each of the compounds (N1), (N2), and (N3) in the first embodiment.

### <Thiol Compound XS>

The polymerizable composition of the second embodiment may further contain a thiol compound XS, which is a thiol compound other than the polythiol compound contained in the above-described polythiol composition.

The thiol compound XS is preferably at least one selected from the group consisting of methanedithiol, 1,2-ethanedithiol, 1,2,3-propanetrithiol, pentaerythritol tetrakis(2-mercaptoacetate), pentaerythritol tetrakis(3-mercaptopropionate), tetrakis(mercaptomethylthiomethyl)methane, tetrakis(2-mercaptoethylthiomethyl)methane, tetrakis(3-mercaptopropylthiomethyl)methane, bis(2-mercaptoethyl) sulfide, bis(2,3-dimercaptopropyl) sulfide, 2,5-dimercaptomethyl-1,4-dithiane, 2,5-dimercapto-1,4-dithiane, 2,5-dimercaptomethyl-2,5-dimethyl-1,4-dithiane, 1,1,3,3-tetrakis(mercaptomethylthio)propane, 1,1,2,2-tetrakis(mercaptomethylthio)ethane, and 4,6-bis(mercaptomethylthio)-1,3-dithiane.

The polymerizable composition of the second embodiment may also contain other components in addition to the polythiol compound and the polyiso(thio)cyanate compound, for the purpose of improving various physical properties of the resin, operability, polymerization reactivity of the polymerizable composition, and the like.

Examples of the other components include a polymerization catalyst, an internal mold release agent, a resin modifier, a chain extender, a crosslinking agent, a radical scavenger, a light stabilizer, a UV absorber, an antioxidant, an oil-soluble dye, a filler, an adhesion improver, an antimicrobial agent, an antistatic agent, a dye, a fluorescent brightener, a fluorescent pigment, and a blue ink agent such as an inorganic pigment.

Examples of the polymerization catalyst include tertiary amine compounds, inorganic and organic acid salts thereof, metal compounds, quaternary ammonium salts, and organic sulfonic acids.

As the internal mold release agent, an acidic phosphoric acid ester can be used. Examples of the acidic phosphoric acid ester include phosphoric acid monoesters and phosphoric acid diesters, and these may be used singly, or in combination of two or more kinds thereof.

Examples of the resin modifier include episulfide compounds, alcohol compounds, amine compounds, epoxy compounds, organic acids and anhydrides thereof, and olefin compounds containing a (meth)acrylate compound or the like.

The polymerizable composition of the second embodiment can be obtained by mixing the above-described components.

### <<Molded Body>>

The molded body of the second embodiment includes the resin of the second embodiment.

The resin of the second embodiment includes a cured product of the polymerizable composition of the second embodiment.

A method of producing the molded body of the second embodiment is not particularly limited, and one example of a preferred production method is cast polymerization. First, the polymerizable composition is injected between casting molds held by a gasket, a tape, or the like. In this process, depending on the physical properties required for the plastic lens to be obtained, it is often preferred to perform, for example, a defoaming treatment under reduced pressure, and a filtration treatment under pressure, reduced pressure, or the like, if necessary.

The polymerization conditions are not limited since they are greatly variable depending on the constitution of the polymerizable composition, the type and the amount of a catalyst to be used, the shape of the molds, and the like, and the polymerization is performed, for example, at a temperature of from -50°C to 150°C over a period of from 1 hour to 50 hours. In some cases, it is preferred to maintain or gradually increase the temperature in a range of from 10°C to 150°C and thereby cure the polymerizable composition for 1 hour to 48 hours.

If necessary, the molded body may be subjected to a treatment such as annealing. The treatment such as annealing is performed in a range of usually from 50°C to 150°C, preferably from 90°C to 140°C, more preferably from 100°C to 130°C.

### [Application]

The resin obtained from the polymerizable composition of the second embodiment can be used as a material for producing molded bodies of various shapes by changing the type of the molds used for cast polymerization.

### <<Optical Material>>

The optical material of the second embodiment contains the resin of the second embodiment.

A molded body obtained from the polymerizable composition of the second embodiment can provide a material in which the yellowness is reduced without impairing the transparency. Further, a molded body obtained from a polymerizable composition containing the polythiol composition of the second embodiment can provide a material that has excellent degree of devitrification as well.

Therefore, the polythiol composition of the second embodiment can be used for various optical materials such as plastic lenses.

### <<Lens>>

The lens of the second embodiment contains the resin of the second embodiment.

As the optical material, a lens is particularly suitable.

Examples of the lens include plastic eyeglass lenses and plastic polarizing lenses.

### [Plastic Eyeglass Lens]

A plastic eyeglass lens using a lens substrate made of the molded body of the second embodiment may have a coating layer applied to one or both sides, if necessary.

The plastic eyeglass lens of the second embodiment includes a lens substrate containing a cured product of the above-described polymerizable composition, and a coating layer.

Specific examples of the coating layer include a primer layer, a hard coat layer, an anti-reflection layer, an anti-fog coating layer, an anti-fouling layer, and a water-repellent layer. These coating layers may each be used singly, or plural coating layers may be used in the form of a multilayer structure. When coating layers are formed on both sides, the same coating layer may be formed on the respective sides, or different coating layers may be formed on the respective sides.

These coating layers may each be used in combination with known additives, such as an infrared absorber for protecting the eyes from infrared rays; a light stabilizer, an antioxidant, or the like for improving the weather resistance of the lens; a photochromic compound, a dye, a pigment, or the like for improving the fashionability of the lens; and an antistatic agent for enhancing the performance of the lens.

With regard to layers to be coated by application, various leveling agents for improving the coating properties may be used.

Further, on an anti-reflection layer, if necessary, an anti-fogging layer, an anti-fouling layer, or a water-repellent layer may be formed.

The second embodiment has been described thus far; however, the above-described constitutions are merely examples of the second embodiment, and various other constitutions can be adopted within a range that does not impair the effects of the second embodiment.

### EXAMPLES

First, examples of the first embodiment of the disclosure will be described; however, the first embodiment of the disclosure is not limited to the below-described Examples.

It is noted here that, unless otherwise specified, "part(s)" is based on mass.

### <Methods of Evaluating Plastic Lens>

In each of the below-described Examples and Comparative Examples, a plastic lens as a resin was evaluated as follows.

The results are as shown in Table 1.

### • Yellowness (Yellow Index (also referred to as "YI"))

A resin was prepared as a circular plastic flat plate of 9 mm in thickness and 75 mm in diameter, and the YI thereof was measured using a spectrophotometer CM-5 manufactured by Konica Minolta, Inc.

The measurement was performed under a transmission condition.

The smaller the YI, the lower is the yellowness of the plastic flat plate, and the larger the YI, the higher is the yellowness.

### • Heat Resistance

A resin test piece of 10 mm in length, 10 mm in width, and 2.5 mm in thickness was prepared. The glass transition temperature (Tg) thereof was measured by a TMA penetration method (load: 50 g, pin tip: 0.5 mmφ, heating rate: 10°C/min) using a thermomechanical analyzer TMA-60 manufactured by Shimadzu Corporation, and the measured value was used as an index of the heat resistance.

The higher the glass transition temperature (Tg), the superior is the heat resistance.

### [Example 1]

### <Production of Polythiol Composition>

A polythiol composition was produced by changing the conditions for thiuronium chlorination reaction using thiourea from "at 110°C for 9 hours" to "at 100°C for 6 hours" in Example 1 of JP-A No. H7-252207. The details thereof are described below.

To a reactor, 89.3 parts by mass of 2-mercaptoethanol, 44.6 parts by mass of deaerated water, and 0.58 parts by mass of a 31%-by-mass aqueous sodium hydroxide solution were added. To this reactor, 107.7 parts by mass of epichlorohydrin was added dropwise at a temperature of from 9°C to 11°C over a period of 4.5 hours, and this was followed by 60-minute stirring. Subsequently, to the reactor, 262.3 parts by mass of a 17.3%-by-mass aqueous sodium sulfide solution was added dropwise at a temperature of from 25°C to 30°C over a period of 1.0 hour, and this was followed by 180-minute stirring. Thereafter, 484.6 parts by mass of 35.0%-by-mass hydrochloric acid was added, and then 214.0 parts by mass of thiourea having a purity of 99.30% by mass was added, after which the resultant was stirred at 100°C for 6 hours to perform a thiuronium chlorination reaction, whereby a liquid containing a thiuronium salt was obtained.

After cooling the thus obtained liquid containing a thiuronium salt to 50°C, 373.0 parts by mass of toluene was added thereto, the liquid was cooled to 35°C, and 347.2 parts by mass of a 25%-by-mass aqueous ammonia solution was subsequently added thereto at a temperature of from 35°C to 40°C over a period of 30 minutes, after which the resultant was stirred at a temperature of from 60°C to 61°C for 1 hour to perform a hydrolysis reaction, whereby a toluene solution containing a polythiol component T, which is at least one selected from the group consisting of 4,8-dimercaptomethyl-1,11-dimercapto-3,6,9-trithiaundecane (hereinafter, also referred to as "compound T1"), 4,7-dimercaptomethyl-1,11-dimercapto-3,6,9-trithiaundecane (hereinafter, also referred to as "compound T2"), and 5,7-dimercaptomethyl-1,11-dimercapto-3,6,9-trithiaundecane (hereinafter, also referred to as "compound T3"), as a main component was obtained. From this toluene solution, an aqueous phase was removed by a liquid separation operation. Then, this toluene solution was acid-washed with an addition of 250.0 parts by mass of 4%-by-mass hydrochloric acid at a temperature of from 35°C to 40°C for 30 minutes, and an operation of acid-washing the toluene solution with an addition of 125.0 parts by mass of 35%-by-mass hydrochloric acid at a temperature of from 35°C to 40°C for 30 minutes was subsequently performed twice, after which an operation of washing the toluene solution with an addition of 125.0 parts by mass of deaerated water at a temperature of from 35°C to 45°C for 15 minutes was performed five times. From the thus washed toluene solution, toluene and a trace amount of water were removed under reduced pressure with heating, and the resultant was then vacuum-filtered through a 3.0-µm PTFE-type membrane filter, whereby 196.7 parts by mass of a polythiol composition containing the polythiol component T as a main component (hereinafter, also referred to as "polythiol composition of Example 1") was obtained.

### <High-Performance Liquid Chromatography (HPLC) Measurement of Polythiol Composition>

For the thus obtained polythiol composition, high-performance liquid chromatography (HPLC) measurement was performed under the above-described measurement conditions A.

Table 1 shows the following as determined by the above-described HPLC of the polythiol composition:
the peak area ratio of the polythiol component T1 (specifically, the ratio of a total peak area of the compounds T1, T2, and T3 with respect to a total peak area of the compounds contained in the polythiol composition);
the peak area ratio of the component X (specifically, the ratio of the peak area of the component X with respect to a total peak area of the compounds contained in the polythiol composition); and
the peak area ratio of the component Y (specifically, the ratio of the peak area of the component Y with respect to a total peak area of the compounds contained in the polythiol composition).

In addition, Table 1 shows the peak area ratio [T1/T2/T3] in the polythiol component T. The peak area ratio [T1/T2/T3] in the polythiol component T means a ratio of the peak areas of the respective compounds T1, T2, and T3 when the peak area of the polythiol component T (i.e., a total peak area of the compounds T1, T2, and T3) is taken as 100.

Further, Table 1 shows the ratio [component Y (area%)/component X (area%)], i.e., a ratio of the peak area of the component Y with respect to the peak area of the component X.

It is noted here that the component X is a polythiol compound having a retention time of from 30.0 minutes to 40.0 minutes and a molecular weight of 426. The molecular formula of the component X is estimated to be C₁₂H₂₆S₈.

The component Y is a polythiol compound having a retention time of from 60.0 minutes to 70.0 minutes and a molecular weight of 532. The molecular formula of the component Y is estimated to be C₁₅H₃₂S₁₀.

### <Production and Evaluation of Plastic Lens>

The following materials were mixed and dissolved at 20°C: 51 parts by mass of *m-*xylylene diisocyanate; 0.015 parts by mass of dibutyl tin dichloride as a curing catalyst; and 0.10 parts by mass of ZELEC UN (trade name, manufactured by Stepan Company; acidic phosphate) as a mold release agent. Subsequently, 49 parts by mass of the polythiol composition of Example 1 was added to and mixed with the resultant to obtain a polymerizable composition as a homogeneous liquid.

The thus obtained polymerizable composition was defoamed at 600 Pa for 1 hour, and then filtered through a 1-µm TEFLON (registered trademark) filter. The thus filtered polymerizable composition was injected between a pair of glass molds immobilized by a tape, and this pair of glass molds was subsequently placed in an oven, after which the oven internal temperature was set at 10°C. Next, the oven internal temperature was raised from 10°C to 120°C over a period of 38 hours. By this process, monomers (polyisocyanate compound and polythiol composition) contained in the defoamed polymerizable composition were polymerized to form a plastic lens containing a thiourethane resin (i.e., a cured product of the polymerizable composition) between the pair of glass molds.

Thereafter, the inside of the oven was cooled, and the pair of glass molds was subsequently taken out of the oven, after which the plastic lens was removed from the pair of glass molds.

For the thus obtained plastic lens, the above-described evaluations were performed.

The results thereof are shown in Table 1.

### [Example 2] (Salt Formation: 0.50 eq)

The above-obtained polythiol composition of Example 1 was made into a salt using 0.50 eq of sodium hydroxide with respect to the SH functional groups in the polythiol composition to obtain a polythiol composition of Example 2. The details thereof are described below.

To a reactor, 100.0 parts by mass of the polythiol composition obtained in Example 1 which contained the polythiol component T as a main component, and 250.0 parts by mass of toluene were added at 25°C to obtain a toluene solution. To the thus obtained toluene solution, 70.6 parts by mass of a 30.9%-by-mass aqueous sodium hydroxide solution (specifically, an aqueous solution containing 0.50 eq of sodium hydroxide with respect to the SH functional groups in the polythiol composition) was added dropwise, and this was followed by 15-minute stirring at a temperature of from 37°C to 40°C. To the resultant, 37.9 parts by mass of deaerated water was added, and this was followed by 15-minute stirring at a temperature of from 37°C to 40°C to extract the components soluble in the aqueous layer side. Subsequently, filtration was performed through a 7-µm filter paper. From the resulting filtrate, an aqueous layer was separated by a liquid separation operation to obtain a toluene layer. The thus obtained toluene layer was washed with stirring at a temperature of from 37°C to 40°C with an addition of 73.9 parts by mass of 35%-by-mass hydrochloric acid. Thereafter, an aqueous layer was separated by a liquid separation operation to obtain a toluene solution. This toluene solution was washed three times with 37.9 parts by mass of deaerated water at a temperature of from 37°C to 40°C, whereby a toluene solution of the polythiol composition was obtained.

From the thus obtained toluene solution, toluene was distilled away using a rotary evaporator. The thus obtained mixture was sequentially subjected to removal of low-boiling-point components using a vacuum pump and filtration through a 3-micron PTFE membrane filter in this order, whereby 46.7 parts by mass of a polythiol composition containing the polythiol component T as a main component (hereinafter, also referred to as "polythiol composition of Example 2") was obtained.

### <Production and Evaluation of Plastic Lens>

The same operations as in "Production and Evaluation of Plastic Lens" in Example 1 were performed, except that "49 parts by mass of the polythiol composition of Example 1" was changed to 49 parts by mass of the polythiol composition of Example 2.

The results thereof are shown in Table 1.

### [Example 3] (Salt Formation: 0.70 eq)

The above-obtained polythiol composition of Example 1 was made into a salt using 0.70 eq of sodium hydroxide with respect to the SH functional groups in the polythiol composition to obtain a polythiol composition of Example 3. The details thereof are described below.

To a reactor, 200.0 parts by mass of the polythiol composition obtained in Example 1 which contained the polythiol component T as a main component, and 500.0 parts by mass of toluene were added at 25°C to obtain a toluene solution. To the thus obtained toluene solution, 197.7 parts by mass of a 30.9%-by-mass aqueous sodium hydroxide solution (specifically, an aqueous solution containing 0.70 eq of sodium hydroxide with respect to the SH functional groups in the polythiol composition) was added dropwise, and this was followed by 15-minute stirring at a temperature of from 37°C to 40°C. To the resultant, 106.1 parts by mass of deaerated water was added, and this was followed by 15-minute stirring at a temperature of from 37°C to 40°C to extract the components soluble in the aqueous layer side. Subsequently, filtration was performed through a 7-µm filter paper. From the resulting filtrate, an aqueous layer was separated by a liquid separation operation to obtain a toluene layer. The thus obtained toluene layer was washed with stirring at a temperature of from 37°C to 40°C with an addition of 206.8 parts by mass of 35%-by-mass hydrochloric acid. Thereafter, an aqueous layer was separated by a liquid separation operation to obtain a toluene solution. This toluene solution was washed three times with 106.1 parts by mass of deaerated water at a temperature of from 37°C to 40°C, whereby a toluene solution of the polythiol composition was obtained.

From the thus obtained toluene solution, toluene was distilled away using a rotary evaporator. The thus obtained mixture was sequentially subjected to removal of low-boiling-point components using a vacuum pump and filtration through a 3-micron PTFE membrane filter in this order, whereby 57.1 parts by mass of a polythiol composition containing the polythiol component T as a main component (hereinafter, also referred to as "polythiol composition of Example 3") was obtained.

### <Production and Evaluation of Plastic Lens>

The same operations as in "Production and Evaluation of Plastic Lens" in Example 1 were performed, except that "49 parts by mass of the polythiol composition of Example 1" was changed to 49 parts by mass of the polythiol composition of Example 3.

The results thereof are shown in Table 1.

**[Table 1]**

| | | Example 1 | Example 2 | Comparative Example 1 |
|---|---|---|---|---|
| Polythiol composition | Polythiol component T (=T1+T2+T3) (area%) | 85.37 | 83.24 | 78.45 |
| | Peak area ratio in polythiol component T (T1/T2/T3) | 85.0/14.7/0.3 | 81.8/17.4/0.8 | 79.1/19.5/1.4 |
| | Component X (area%) | 2.07 | 3.95 | 5.59 |
| | Component Y (area%) | 1.45 | 2.38 | 2.79 |
| | Peak area ratio [Component Y/Component X] | 0.70 | 0.60 | 0.50 |
| Plastic lens | Yl (transmission) | 2.0 | 2.7 | 3.5 |
| | Tg (°C) | 103.44 | 101.17 | 97.90 |

As shown in Table 1, in the plastic lenses (i.e., resins) of Examples 1 and 2 in which the peak area of the component X was 5.00 or less and the peak area of the component Y was 2.50 or less with respect to a total peak area of 100 of the compounds contained in the respective polythiol compositions, the yellowness (YI) was reduced and the heat resistance (specifically, Tg) was improved.

In contrast, in the plastic lens (i.e., resin) of Comparative Example 1 in which the peak area of the component X was more than 5.00 and the peak area of the component Y was more than 2.50 with respect to a total peak area of 100 of the compounds contained in the polythiol composition, the yellowness (YI) was increased and the heat resistance (specifically, Tg) was reduced.

### [Example 1X, Example 2X, and Comparative Example 1X]

As Example 1X, Example 2X, and Comparative Example 1X, the same operations were performed as in Example 1, Example 2, and Comparative Example 1, respectively, except that the production of a plastic lens was changed as follows, and results similar to those of Example 1, Example 2, and Comparative Example 1 (Table 1) were obtained.

### -Changes from Example 1, Example 2, and Comparative Example 1-

In each of Example 1, Example 2, and Comparative Example 1, *m*-xylylene diisocyanate (51 parts by mass) was used in the production of the respective plastic lenses; however, in each of Example 1X, Example 2X, and Comparative Example 1X, XDI (51 parts by mass) was changed to an XDI composition X1 (in such an amount that the amount of *m-*xylylene diisocyanate contained therein was 51 parts by mass) as the above-described XDI composition.

The XDI composition X1 was produced by adding trace amounts of the compounds (N1), (N2), and (N3) to XDI serving as a main component, and then mixing these materials.

As a result of performing gas chromatography measurement for the XDI composition X1 under each of the above-described GC conditions 1 and 2, it was found that:
the peak area of the compound (N1) was 0.20 ppm or more (specifically, 600 ppm) with respect to a peak area of xylylene diisocyanate of 1;
the peak area of the compound (N2) was 0.05 ppm or more (specifically, 18 ppm) with respect to a peak area of xylylene diisocyanate of 1; and
the peak area of the compound (N3) was 0.10 ppm or more (specifically, 100 ppm) with respect to a peak area of xylylene diisocyanate of 1.

Next, examples of the second embodiment of the disclosure will be described; however, the second embodiment of the disclosure is not limited to the below-described Examples.

It is noted here that, unless otherwise specified, "part(s)" is based on mass.

### <Methods of Evaluating Plastic Lens>

In each of the below-described Examples and Comparative Examples, a plastic lens as a resin was evaluated as follows.

The results are as shown in Table 2.

### • Yellowness (Yellow Index (also referred to as "YI"))

A resin was prepared as a circular plastic flat plate of 9 mm in thickness and 75 mm in diameter, and the YI thereof was measured using a spectrophotometer CM-5 manufactured by Konica Minolta, Inc.

The measurement was performed under a transmission condition.

The smaller the YI, the lower the yellowness of the plastic flat plate, and the larger the YI, the higher the yellowness.

### • Heat Resistance

A resin test piece of 10 mm in length, 10 mm in width, and 2.5 mm in thickness was prepared. The glass transition temperature (Tg) thereof was measured by a TMA penetration method (load: 50 g, pin tip: 0.5 mmφ, heating rate: 10°C/min) using a thermomechanical analyzer TMA-60 manufactured by Shimadzu Corporation, and the measured value was used as an index of the heat resistance.

The higher the glass transition temperature (Tg), the superior the heat resistance is.

### [Example 101]

### <Production of Polythiol Composition>

A polythiol composition was produced by changing the conditions for thiuronium chlorination reaction using thiourea from "at 110°C for 9 hours" to "at 105°C for 7 hours" in Example 1 of JP-A No. H7-252207. The details thereof are described below.

To a reactor, 89.3 parts by mass of 2-mercaptoethanol, 44.6 parts by mass of deaerated water, and 0.58 parts by mass of a 31%-by-mass aqueous sodium hydroxide solution were added. To this reactor, 107.7 parts by mass of epichlorohydrin was added dropwise at a temperature of from 9°C to 11°C over a period of 4.5 hours, and this was followed by 60-minute stirring. Subsequently, to the reactor, 262.3 parts by mass of a 17.3%-by-mass aqueous sodium sulfide solution was added dropwise at a temperature of from 25°C to 30°C over a period of 1.0 hour, and this was followed by 180-minute stirring. Thereafter, 484.6 parts by mass of 35.0%-by-mass hydrochloric acid was added, and then 214.0 parts by mass of thiourea having a purity of 99.30% by mass was added, after which the resultant was stirred at 105°C for 7 hours to perform a thiuronium chlorination reaction, whereby a liquid containing a thiuronium salt was obtained.

After cooling the thus obtained liquid containing a thiuronium salt to 50°C, 373.0 parts by mass of toluene was added thereto, the liquid was cooled to 35°C, and 347.2 parts by mass of a 25%-by-mass aqueous ammonia solution was subsequently added thereto at a temperature of from 35°C to 40°C over a period of 30 minutes, after which the resultant was stirred at a temperature of from 60°C to 61°C for 1 hour to perform a hydrolysis reaction, whereby a toluene solution containing a polythiol component T, which is at least one selected from the group consisting of 4,8-dimercaptomethyl-1,11-dimercapto-3,6,9-trithiaundecane (hereinafter, also referred to as "compound T1"), 4,7-dimercaptomethyl-1,11-dimercapto-3,6,9-trithiaundecane (hereinafter, also referred to as "compound T2"), and 5,7-dimercaptomethyl-1,11-dimercapto-3,6,9-trithiaundecane (hereinafter, also referred to as "compound T3"), as a main component was obtained. From this toluene solution, an aqueous phase was removed by a liquid separation operation. Then, this toluene solution was acid-washed with an addition of 250.0 parts by mass of 4%-by-mass hydrochloric acid at a temperature of from 35°C to 40°C for 30 minutes, and an operation of acid-washing the toluene solution with an addition of 125.0 parts by mass of 35%-by-mass hydrochloric acid at a temperature of from 35°C to 40°C for 30 minutes was subsequently performed twice, after which an operation of washing the toluene solution with an addition of 125.0 parts by mass of deaerated water at a temperature of from 35°C to 45°C for 15 minutes was performed five times. From the thus washed toluene solution, toluene and a trace amount of water were removed under reduced pressure with heating, and the resultant was then vacuum-filtered through a 3.0-µm PTFE-type membrane filter, whereby 196.7 parts by mass of a polythiol composition containing the polythiol component T as a main component (hereinafter, also referred to as "polythiol composition of Example 101") was obtained.

### <High-Performance Liquid Chromatography (HPLC) Measurement of Polythiol Composition>

For the thus obtained polythiol composition, high-performance liquid chromatography (HPLC) measurement was performed under the above-described measurement conditions A.

Table 2 shows the following as determined by the above-described HPLC of the polythiol composition:
the peak area ratio of the polythiol component T (specifically, the total peak area (area%) of the compounds T1, T2, and T3 with respect to a total peak area of 100 of the compounds contained in the polythiol composition);
the peak area ratio of the component W (specifically, the peak area (area%) of the component W with respect to a total peak area of 100 of the compounds contained in the polythiol composition);
the peak area ratio of the component V (specifically, the peak area (area%) of the component V with respect to a total peak area of 100 of the compounds contained in the polythiol composition);
the peak area ratio of the component X (specifically, the peak area (area%) of the component X with respect to a total peak area of 100 of the compounds contained in the polythiol composition);
the peak area ratio of the component Y (specifically, the ratio of the peak area of the component Y with respect to a total peak area of 100 of the compounds contained in the polythiol composition); and
the peak area ratio of the component U (specifically, the peak area (area%) of the component U with respect to a total peak area of 100 of the compounds contained in the polythiol composition).

In addition, Table 2 shows the peak area ratio [T1/T2/T3] in the polythiol component T. The peak area ratio [T1/T2/T3] in the polythiol component T means a ratio of the peak areas of the respective compounds T1, T2, and T3 when the peak area of the polythiol component T (i.e., a total peak area of the compounds T1, T2, and T3) is taken as 100.

Further, Table 2 shows:
the ratio [component V/component W] (i.e., a ratio of the peak area of the component V with respect to the peak area of the component W);
the ratio [component V/polythiol component T] (i.e., a ratio of the peak area of the component V with respect to the peak area of the polythiol component T); and
the ratio [component X/component Y] (i.e., a ratio of the peak area of the component X with respect to the peak area of the component Y).

It is noted here that the component W is a polythiol compound having a retention time of from 8.7 minutes to 9.7 minutes and a molecular weight of 350. The molecular formula of the component W is estimated to be C₁₀H₂₂OS₆.

The component V is a polythiol compound having a retention time of from 11.0 minutes to 13.5 minutes and a molecular weight of 260. The molecular formula of the component V is estimated to be C₇H₁₆S₅.

The component X is a polythiol compound having a retention time of from 30.0 minutes to 40.0 minutes and a molecular weight of 426. The molecular formula of the component X is estimated to be C₁₂H₂₆S₈.

The component Y is a polythiol compound having a retention time of from 60.0 minutes to 70.0 minutes and a molecular weight of 532. The molecular formula of the component Y is estimated to be C₁₅H₃₂S₁₀.

The component U is a polythiol compound having a retention time of from 40.0 minutes to 49.0 minutes and a molecular weight of 472. The molecular formula of the component U is estimated to be C₁₃H₂₈S₉.

### <Production and Evaluation of Plastic Lens>

The following materials were mixed and dissolved at 20°C: 51 parts by mass of *m-*xylylene diisocyanate; 0.015 parts by mass of dibutyl tin dichloride as a curing catalyst; and 0.10 parts by mass of ZELEC UN (trade name, manufactured by Stepan Company; acidic phosphate) as a mold release agent. Subsequently, 49 parts by mass of the polythiol composition of Example 101 was added to and mixed with the resultant to obtain a polymerizable composition as a homogeneous liquid.

The thus obtained polymerizable composition was defoamed at 600 Pa for 1 hour, and then filtered through a 1-µm TEFLON (registered trademark) filter. The thus filtered polymerizable composition was injected between a pair of glass molds immobilized by a tape, and this pair of glass molds was subsequently placed in an oven, after which the oven internal temperature was set at 10°C. Next, the oven internal temperature was raised from 10°C to 120°C over a period of 38 hours. By this process, monomers (polyisocyanate compound and polythiol composition) contained in the defoamed polymerizable composition were polymerized to form a plastic lens containing a thiourethane resin (i.e., a cured product of the polymerizable composition) between the pair of glass molds.

Thereafter, the inside of the oven was cooled, and the pair of glass molds was subsequently taken out of the oven, after which the plastic lens was removed from the pair of glass molds.

For the thus obtained plastic lens, the above-described evaluations were performed.

The results thereof are shown in Table 2.

### [Example 102]

### <Production of Polythiol Composition>

The above-obtained polythiol composition of Example 101 was made into a salt using 0.50 eq of sodium hydroxide with respect to the SH functional groups in the polythiol composition to obtain a polythiol composition of Example 102. The details thereof are described below.

To a reactor, 150.0 parts by mass of the polythiol composition obtained in Example 101 which contained the polythiol component T as a main component, and 375.0 parts by mass of toluene were added at 25°C to obtain a toluene solution. To the thus obtained toluene solution, 106.6 parts by mass of a 30.7%-by-mass aqueous sodium hydroxide solution (specifically, an aqueous solution containing 0.50 eq of sodium hydroxide with respect to the SH functional groups in the polythiol composition) was added dropwise, and this was followed by 15-minute stirring at a temperature of from 37°C to 40°C. To the resultant, 57.2 parts by mass of deaerated water was added, and this was followed by 15-minute stirring at a temperature of from 37°C to 40°C to extract the components soluble in the aqueous layer side. Subsequently, filtration was performed through a 7-µm filter paper. From the resulting filtrate, a toluene layer was separated by a liquid separation operation to obtain an aqueous layer. The thus obtained aqueous layer was washed with stirring at a temperature of from 37°C to 40°C by adding thereto 150.0 parts by mass of toluene and 110.8 parts by mass of 35%-by-mass hydrochloric acid. Thereafter, the aqueous layer was separated by a liquid separation operation to obtain a toluene solution. This toluene solution was washed three times with 57.2 parts by mass of deaerated water at a temperature of from 37°C to 40°C, whereby a toluene solution of the polythiol composition was obtained.

From the thus obtained toluene solution, toluene was distilled away using a rotary evaporator. The thus obtained mixture was sequentially subjected to removal of low-boiling-point components using a vacuum pump and filtration through a 3-micron PTFE membrane filter in this order, whereby 71.1 parts by mass of a polythiol composition containing the polythiol component T as a main component (hereinafter, also referred to as "polythiol composition of Example 102") was obtained.

### <Production and Evaluation of Plastic Lens>

The same operations as in "Production and Evaluation of Plastic Lens" in Example 101 were performed, except that "49 parts by mass of the polythiol composition of Example 101" was changed to 49 parts by mass of the polythiol composition of Example 102.

The results thereof are shown in Table 2.

### [Example 103]

The above-obtained polythiol composition of Example 101 was made into a salt using 0.70 eq of sodium hydroxide with respect to the SH functional groups in the polythiol composition to obtain a polythiol composition of Example 103. The details thereof are described below.

To a reactor, 150.0 parts by mass of the polythiol composition obtained in Example 101 which contained the polythiol component T as a main component, and 375.0 parts by mass of toluene were added at 25°C to obtain a toluene solution. To the thus obtained toluene solution, 149.2 parts by mass of a 30.7%-by-mass aqueous sodium hydroxide solution (specifically, an aqueous solution containing 0.70 eq of sodium hydroxide with respect to the SH functional groups in the polythiol composition) was added dropwise, and this was followed by 15-minute stirring at a temperature of from 37°C to 40°C. To the resultant, 80.1 parts by mass of deaerated water was added, and this was followed by 15-minute stirring at a temperature of from 37°C to 40°C to extract the components soluble in the aqueous layer side. Subsequently, filtration was performed through a 7-µm filter paper. From the resulting filtrate, a toluene layer was separated by a liquid separation operation to obtain an aqueous layer. The thus obtained aqueous layer was washed with stirring at a temperature of from 37°C to 40°C by adding thereto 150.0 parts by mass of toluene and 155.1 parts by mass of 35%-by-mass hydrochloric acid. Thereafter, the aqueous layer was separated by a liquid separation operation to obtain a toluene solution. This toluene solution was washed three times with 80.1 parts by mass of deaerated water at a temperature of from 37°C to 40°C, whereby a toluene solution of the polythiol composition was obtained.

From the thus obtained toluene solution, toluene was distilled away using a rotary evaporator. The thus obtained mixture was sequentially subjected to removal of low-boiling-point components using a vacuum pump and filtration through a 3-micron PTFE membrane filter in this order, whereby 99.4 parts by mass of a polythiol composition containing the polythiol component T as a main component (hereinafter, also referred to as "polythiol composition of Example 103") was obtained.

### <Production and Evaluation of Plastic Lens>

The same operations as in "Production and Evaluation of Plastic Lens" in Example 101 were performed, except that "49 parts by mass of the polythiol composition of Example 101" was changed to 49 parts by mass of the polythiol composition of Example 103.

The results thereof are shown in Table 1.

### [Example 104]

### <Production of Polythiol Composition>

The above-obtained polythiol composition of Example 101 was made into a salt using 0.50 eq of sodium hydroxide with respect to the SH functional groups in the polythiol composition to obtain a polythiol composition of Example 104. The details thereof are described below.

To a reactor, 100.0 parts by mass of the polythiol composition obtained in Example 101 which contained the polythiol component T as a main component, and 250.0 parts by mass of toluene were added at 25°C to obtain a toluene solution. To the thus obtained toluene solution, 70.6 parts by mass of a 30.9%-by-mass aqueous sodium hydroxide solution (specifically, an aqueous solution containing 0.50 eq of sodium hydroxide with respect to the SH functional groups in the polythiol composition) was added dropwise, and this was followed by 15-minute stirring at a temperature of from 37°C to 40°C. To the resultant, 37.9 parts by mass of deaerated water was added, and this was followed by 15-minute stirring at a temperature of from 37°C to 40°C to extract the components soluble in the aqueous layer side. Subsequently, filtration was performed through a 7-µm filter paper. From the resulting filtrate, an aqueous layer was separated by a liquid separation operation to obtain a toluene layer. The thus obtained toluene layer was washed with stirring at a temperature of from 37°C to 40°C with an addition of 73.9 parts by mass of 35%-by-mass hydrochloric acid. Thereafter, an aqueous layer was separated by a liquid separation operation to obtain a toluene solution. This toluene solution was washed three times with 37.9 parts by mass of deaerated water at a temperature of from 37°C to 40°C, whereby a toluene solution of the polythiol composition was obtained.

From the thus obtained toluene solution, toluene was distilled away using a rotary evaporator. The thus obtained mixture was sequentially subjected to removal of low-boiling-point components using a vacuum pump and filtration through a 3-micron PTFE membrane filter in this order, whereby 46.7 parts by mass of a polythiol composition containing the polythiol component T as a main component (hereinafter, also referred to as "polythiol composition of Example 104") was obtained.

### <Production and Evaluation of Plastic Lens>

The same operations as in "Production and Evaluation of Plastic Lens" in Example 101 were performed, except that "49 parts by mass of the polythiol composition of Example 101" was changed to 49 parts by mass of the polythiol composition of Example 104.

The results thereof are shown in Table 2.

### [Comparative Example 101]

The above-obtained polythiol composition of Example 101 was made into a salt using 0.70 eq of sodium hydroxide with respect to the SH functional groups in the polythiol composition to obtain a polythiol composition of Comparative Example 101. The details thereof are described below.

To a reactor, 200.0 parts by mass of the polythiol composition obtained in Example 101 which contained the polythiol component T as a main component, and 500.0 parts by mass of toluene were added at 25°C to obtain a toluene solution. To the thus obtained toluene solution, 197.7 parts by mass of a 30.9%-by-mass aqueous sodium hydroxide solution (specifically, an aqueous solution containing 0.70 eq of sodium hydroxide with respect to the SH functional groups in the polythiol composition) was added dropwise, and this was followed by 15-minute stirring at a temperature of from 37°C to 40°C. To the resultant, 106.1 parts by mass of deaerated water was added, and this was followed by 15-minute stirring at a temperature of from 37°C to 40°C to extract the components soluble in the aqueous layer side. Subsequently, filtration was performed through a 7-µm filter paper. From the resulting filtrate, an aqueous layer was separated by a liquid separation operation to obtain a toluene layer. The thus obtained toluene layer was washed with stirring at a temperature of from 37°C to 40°C with an addition of 206.8 parts by mass of 35%-by-mass hydrochloric acid. Thereafter, an aqueous layer was separated by a liquid separation operation to obtain a toluene solution. This toluene solution was washed three times with 106.1 parts by mass of deaerated water at a temperature of from 37°C to 40°C, whereby a toluene solution of the polythiol composition was obtained.

From the thus obtained toluene solution, toluene was distilled away using a rotary evaporator. The thus obtained mixture was sequentially subjected to removal of low-boiling-point components using a vacuum pump and filtration through a 3-micron PTFE membrane filter in this order, whereby 57.1 parts by mass of a polythiol composition containing the polythiol component T as a main component (hereinafter, also referred to as "polythiol composition of Comparative Example 101") was obtained.

### <Production and Evaluation of Plastic Lens>

The same operations as in "Production and Evaluation of Plastic Lens" in Example 101 were performed, except that "49 parts by mass of the polythiol composition of Example 101" was changed to 49 parts by mass of the polythiol composition of Comparative Example 101.

The results thereof are shown in Table 2.

**[Table 2]**

| | | Example 101 | Example 102 | Example 103 | Example 104 | Comparative Example 101 |
|---|---|---|---|---|---|---|
| | Polythiol component T (=T1+T2+T3) (area%) | 84.37 | 86.94 | 87.42 | 83.10 | 78.21 |
| | Peak area ratio in polythiol component T (T1/T2/T3) | 85.00/14.60/0.40 | 87.20/12.80/0.00 | 86.90/13.10/0.00 | 81.70/17.50/0.80 | 79.10/19.60/1.30 |
| | Component W (area%) | 2.02 | 2.69 | 2.38 | 1.50 | 1.27 |
| | Component V (area%) | 2.35 | 1.90 | 1.98 | 2.83 | 3.24 |
| Polythiol composition | Peak area ratio [Component V/Component W] | 1.16 | 0.71 | 0.83 | 1.89 | 2.55 |
| | Peak area ratio [Component V/Polythiol component T] | 0.03 | 0.02 | 0.02 | 0.03 | 0.04 |
| | Component X (area%) | 2.07 | 0.70 | 0.85 | 3.90 | 5.52 |
| | Component Y (area%) | 1.45 | 0.67 | 0.85 | 2.42 | 2.78 |
| | Peak area ratio [Component Y/Component X] | 0.70 | 0.96 | 1.00 | 0.62 | 0.50 |
| | Component U (area%) | 2.99 | 3.63 | 3.50 | 2.35 | 2.02 |
| Plastic lens | Yl | 2.0 | 1.5 | 1.6 | 2.7 | 3.5 |
| | Tg (°C) | 103.0 | 104.5 | 105.8 | 101.1 | 97.8 |

As shown in Table 2, in the plastic lenses (i.e., resins) of Examples 101 to 104 in which the peak area of the component W was 3.50 or less and the peak area of the component V was 3.10 or less with respect to a total peak area of 100 of the compounds contained in the respective polythiol compositions, the yellowness (YI) was reduced and the heat resistance (specifically, Tg) was improved.

In contrast, in the plastic lens (i.e., resin) of Comparative Example 101 in which the peak area of the component V was more than 3.10 with respect to a total peak area of 100 of the compounds contained in the polythiol composition, the yellowness (YI) was increased and the heat resistance (specifically, Tg) was reduced.

### [Example 101X, Example 102X, Example 103X, Example 104X, and Comparative Example 101X]

As Example 101X, Example 102X, Example 103X, Example 104X, and Comparative Example 101X, the same operations were performed as in Example 101, Example 102, Example 103, Example 104, and Comparative Example 101, respectively, except that the production of a plastic lens was changed as follows, and results similar to those of Example 101, Example 102, Example 103, Example 104, and Comparative Example 101 (Table 2) were obtained.

### -Changes from Example 101, Example 102, Example 103, Example 104, and Comparative Example 101-

In each of Example 101, Example 102, Example 103, Example 104, and Comparative Example 101, *m*-xylylene diisocyanate (51 parts by mass) was used in the production of the respective plastic lenses; however, in each of Example 101X, Example 102X, Example 103X, Example 104X, and Comparative Example 101X, XDI (51 parts by mass) was changed to an XDI composition X101 (in such an amount that the amount of *m-*xylylene diisocyanate contained therein was 51 parts by mass) as the above-described XDI composition.

The XDI composition X101 was produced by adding trace amounts of the compounds (N1), (N2), and (N3) to XDI serving as a main component, and then mixing these materials.

As a result of performing gas chromatography measurement for the XDI composition X101 under each of the above-described GC conditions 1 and 2, it was found that:
the peak area of the compound (N1) was 0.20 ppm or more (specifically, 600 ppm) with respect to a peak area of xylylene diisocyanate of 1;
the peak area of the compound (N2) was 0.05 ppm or more (specifically, 18 ppm) with respect to a peak area of xylylene diisocyanate of 1; and
the peak area of the compound (N3) was 0.10 ppm or more (specifically, 100 ppm) with respect to a peak area of xylylene diisocyanate of 1.

The disclosure of Japanese Patent Application No. 2023-191755 filed on November 9, 2023, and the disclosure of Japanese Patent Application No. 2024-094653 filed on June 11, 2024 are hereby incorporated by reference in their entirety.

All the documents, patent applications and technical standards that are described in the present specification are hereby incorporated by reference to the same extent as if each individual document, patent application or technical standard is concretely and individually described to be incorporated by reference.

## Claims

1. A polythiol composition, comprising:
a polythiol component T, which is at least one selected from the group consisting of 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane;
a component X, which has a retention time of from 30.0 minutes to 40.0 minutes in a high-performance liquid chromatography measurement performed under the following measurement conditions A; and
a component Y, for which the retention time is from 60.0 minutes to 70.0 minutes,
wherein, in the high-performance liquid chromatography measurement, the polythiol component T has a peak area of 80.00 or more with respect to a total peak area of 100 of compounds contained in the polythiol composition, the component X has a peak area of 5.00 or less with respect to the total peak area of 100, and the component Y has a peak area of 2.50 or less with respect to the total peak area of 100:
- Measurement Conditions A-
YMC-Pack (registered trademark) ODS-A manufactured by YMC Co., Ltd. (particle size S: 5 µm, column shape: Φ6 mm × 150 mm) is used as a column;
a mixed solution of acetonitrile / 0.01mol/L-aqueous potassium dihydrogen phosphate solution = 60/40 (vol/vol) is used as a mobile phase;
a mixed solution of 160 mg of the polythiol composition and 10 mL of acetonitrile is used as a measurement solution;
a UV detector having a measurement wavelength of 230 nm is used as a detector;
a column temperature is set at 40°C;
a flow rate is set at 1.0 mL/min; and
an injection amount is set at 2 µL.

2. The polythiol composition according to claim 1, wherein:
the component X is a polythiol compound having a molecular weight of 426, and
the component Y is a polythiol compound having a molecular weight of 532.

3. The polythiol composition according to claim 1, wherein:
the peak area of the component X is 1.00 or more, and
the peak area of the component Y is 1.00 or more.

4. The polythiol composition according to claim 1, wherein a ratio of the peak area of the component Y to the peak area of the component X is from 0.40 to 0.90.

5. A polythiol composition, comprising:
a polythiol component T, which is at least one selected from the group consisting of 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane;
a component W, which has a retention time of from 8.7 minutes to 9.7 minutes in a high-performance liquid chromatography measurement performed under the following measurement conditions A; and
a component V, for which the retention time is from 11.0 minutes to 13.5 minutes,
wherein, in the high-performance liquid chromatography measurement, the polythiol component T has a peak area of 75.00 or more with respect to a total peak area of 100 of compounds contained in the polythiol composition, the component W has a peak area of 3.50 or less with respect to the total peak area of 100, and the component V has a peak area of 3.10 or less with respect to the total peak area of 100:
- Measurement Conditions A-
YMC-Pack (registered trademark) ODS-A manufactured by YMC Co., Ltd. (particle size S: 5 µm, column shape: Φ6 mm × 150 mm) is used as a column;
a mixed solution of acetonitrile / 0.01mol/L-aqueous potassium dihydrogen phosphate solution = 60/40 (vol/vol) is used as a mobile phase;
a mixed solution of 160 mg of the polythiol composition and 10 mL of acetonitrile is used as a measurement solution;
a UV detector having a measurement wavelength of 230 nm is used as a detector;
a column temperature is set at 40°C;
a flow rate is set at 1.0 mL/min; and
an injection amount is set at 2 µL.

6. The polythiol composition according to claim 5, wherein:
the component W is a polythiol compound having a molecular weight of 350, and
the component V is a polythiol compound having a molecular weight of 260.

7. The polythiol composition according to claim 5, wherein:
the peak area of the component W is 1.30 or more with respect to the total peak area of 100, and
the peak area of the component V is 1.00 or more with respect to the total peak area of 100.

8. The polythiol composition according to claim 5, wherein a ratio of the peak area of the component V with respect to the total peak area of 100 to the peak area of the component W with respect to the total peak area of 100 is from 0.10 to 2.80.

9. The polythiol composition according to claim 5, further comprising at least one of a component X, which has a retention time of from 30.0 minutes to 40.0 minutes in the high-performance liquid chromatography measurement performed under the measurement conditions A, or a component Y, for which the retention time is from 60.0 minutes to 70.0 minutes.

10. A polymerizable composition, comprising:
the polythiol composition according to any one of claims 1 to 9; and
a polyiso(thio)cyanate compound.

11. The polymerizable composition according to claim 10, further comprising a thiol compound XS, which is a thiol compound other than polythiol compounds contained in the polythiol composition.

12. The polymerizable composition according to claim 11, wherein the thiol compound XS is at least one selected from the group consisting of methanedithiol, 1,2-ethanedithiol, 1,2,3-propanetrithiol, pentaerythritol tetrakis(2-mercaptoacetate), pentaerythritol tetrakis(3-mercaptopropionate), tetrakis(mercaptomethylthiomethyl)methane, tetrakis(2-mercaptoethylthiomethyl)methane, tetrakis(3-mercaptopropylthiomethyl)methane, bis(2-mercaptoethyl) sulfide, bis(2,3-dimercaptopropyl) sulfide, 2,5-dimercaptomethyl-1,4-dithiane, 2,5-dimercapto-1,4-dithiane, 2,5-dimercaptomethyl-2,5-dimethyl-1,4-dithiane, 1,1,3,3-tetrakis(mercaptomethylthio)propane, 1,1,2,2-tetrakis(mercaptomethylthio)ethane, and 4,6-bis(mercaptomethylthio)-1,3-dithiane.

13. The polymerizable composition according to claim 10, wherein the polyiso(thio)cyanate compound comprises at least one selected from the group consisting of pentamethylene diisocyanate, hexamethylene diisocyanate, xylylene diisocyanate, isophorone diisocyanate, bis(isocyanatomethyl)cyclohexane, bis(isocyanatocyclohexyl)methane, 2,5-bis(isocyanatomethyl)bicyclo-[2.2.1]-heptane, 2,6-bis(isocyanatomethyl)bicyclo-[2.2.1]-heptane, tolylene diisocyanate, 4,4'-diphenylmethane diisocyanate, and phenylene diisocyanate.

14. The polymerizable composition according to claim 10, comprising a polyiso(thio)cyanate composition comprising the polyiso(thio)cyanate compound,
wherein:
the polyiso(thio)cyanate composition comprises:
xylylene diisocyanate, and
at least one selected from the group consisting of the following compounds (N1), (N2), and (N3);
when the polyiso(thio)cyanate composition comprises the compound (N1), the compound (N1) has a peak area of 0.20 ppm or more with respect to a peak area of xylylene diisocyanate of 1 in a gas chromatography measurement;
when the polyiso(thio)cyanate composition comprises the compound (N2), the compound (N2) has a peak area of 0.05 ppm or more with respect to a peak area of xylylene diisocyanate of 1 in a gas chromatography measurement; and
when the polyiso(thio)cyanate composition comprises the compound (N3), the compound (N3) has a peak area of 0.10 ppm or more with respect to a peak area of xylylene diisocyanate of 1 in a gas chromatography measurement

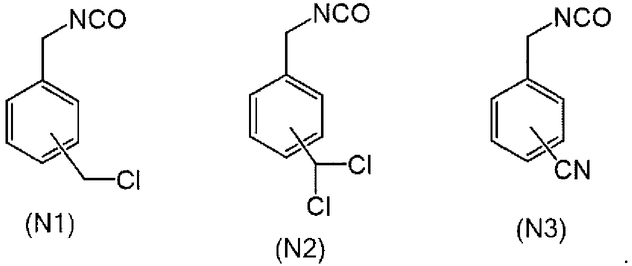

15. A resin, comprising a cured product of the polymerizable composition according to claim 10.

16. A molded body, comprising the resin according to claim 15.

17. An optical material, comprising the resin according to claim 15.

18. A lens, comprising the resin according to claim 15.
